# EUROPÄISCHE PATENTSCHRIFT

(11) **EP 2 035 833 B1**
(45) Veröffentlichungstag und Bekanntmachung des Hinweises auf die Patenterteilung: **11.09.2013**
(21) Anmeldenummer: 08716361.4
(22) Anmeldetag: 07.03.2008
(51) Int. Cl.: G01N 33/569

(54) **BIOCHIP UND VERFAHREN ZUM SELEKTIVEN NACHWEIS VON CHLAMYDIA TRACHOMATIS- INFEKTIONEN**
BIOCHIP AND METHOD FOR THE SELECTIVE IDENTIFICATION OF CHLAMYDIA TRACHOMATIS INFECTIONS
BIOPUCE ET PROCÉDÉ POUR LA DÉTECTION SÉLECTIVE D'INFECTIONS À CHLAMYDIA TRACHOMATIS

(30) Priorität: 07.03.2007 EP 07004665
(43) Veröffentlichungstag der Anmeldung: 18.03.2009
(73) Patentinhaber: Mikrogen GmbH, 82061 Neuried (DE)
(72) Erfinder: SIMNACHER, Ulrike, 89075 Ulm (DE); FORSBACH-BIRK, Vera, 89075 Ulm (DE); ESSIG, Andreas, 89075 Ulm (DE); PFREPPER, Klaus-Ingmar, 81375 München (DE)
(74) Vertreter: Keller, Günter
(86) Internationale Anmeldenummer: PCT/EP2008/001847
(87) Internationale Veröffentlichungsnummer: WO 2008/107203

(56) Entgegenhaltungen:
- WO-A-2004/074318
- WO-A-2007/110700
- WO-A2-02/08267
- SHARMA JYOTIKA ET AL: "Profiling of human antibody responses to Chlamydia trachomatis urogenital tract infection using microplates arrayed with 156 chlamydial fusion proteins" INFECTION AND IMMUNITY, Bd. 74, Nr. 3, März 2006 (2006-03), Seiten 1490-1499, XP002451456 ISSN: 0019-9567 in der Anmeldung erwähnt
- SANCHEZ-CAMPILLO M ET AL: "Identification of immunoreactive proteins of Chlamydia trachomatis by Western blot analysis of a two-dimensional electrophoresis map with patient sera." ELECTROPHORESIS AUG 1999, Bd. 20, Nr. 11, August 1999 (1999-08), Seiten 2269-2279, XP000900035 ISSN: 0173-0835
- BIENDO M ET AL: "LIMITS OF THE MICROIMMUNOFLUORESCENCE TEST AND ADVANTAGES OF IMMUNOBLOTTING IN THE DIAGNOSIS OF CHLAMYDIOSIS" CLINICAL AND DIAGNOSTIC LABORATORY IMMUNOLOGY, AMERICAN SOCIETY FOR MICROBIOLOGY, US, Bd. 3, Nr. 6, November 1996 (1996-11), Seiten 706-709, XP000853580 ISSN: 1071-412X
- MIKROGEN GMBH:[Online] XP002451850 Produktinformation für ein im Jahr 2006 erhältliches Produkt Gefunden im Internet: URL:http://www.mikrogen.de/download/PIRLCY D.pdf>
- "Zur Bedeutung von Chlamydien-Infektionen; recomLine Chlamydia Produkt-Präsentation" MIKROGEN FORUM, Bd. 4, Nr. 15, November 2005 (2005-11), Seiten 1-4, XP002486262
- JORN SIEMER ET AL: 'Chlamydia trachomatis infection as a risk factor for infertility among women in Ghana, West Africa.' THE AMERICAN JOURNAL OF TROPICAL MEDICINE AND HYGIENE Bd. 78, Nr. 2, 01 Februar 2008, Seiten 323 - 327, XP055041534 ISSN: 0002-9637
- V. FORSBACH-BIRK ET AL: 'Identification and evaluation of a combination of chlamydial antigens to support the diagnosis of severe and invasive Chlamydia trachomatis infections' CLINICAL MICROBIOLOGY AND INFECTION Bd. 16, Nr. 8, 01 August 2010, Seiten 1237 - 1244, XP055041532 DOI: 10.1111/j.1469-0691.2009.03041.x ISSN: 1198-743X

## Beschreibung

Die vorliegende Erfindung betrifft ein Verfahren zum selektiven Nachweis von *Chlamydia trachomatis*-Infektionen, worin die Antigene CT017, CT098, CT318-L1P, CT431, CT456-TARP, CT603-TSAP und CT664 zum Nachweis von *Chlamydia trachomatis*-spezifischen Antikörpern in Proben von Säugetieren verwendet werden. Des Weiteren betrifft die Erfindung einen Biochip, der die vorstehend genannten Antigene zum Nachweis der *Chlamydia trachomatis*-spezifischen Antikörper aufweist. Die vorliegende Erfindung betrifft zudem ein Testkit, das zum Nachweis von *Chlamydia trachomatis-*Infektionen geeignet ist.

Chlamydien sind weltweit verbreitete Infektionserreger von erheblicher klinischer und epidemiologischer Relevanz. Es handelt sich um obligat intrazelluläre replizierende Bakterien, die morphologisch und funktionell in zwei unterschiedlichen Zellformen vorliegen (hochinfektiöse, stoffwechselinaktive Elementarkörper und replikationsfähige, intrazelluläre Retikularkörper) und einen typischen Entwicklungszyklus durchlaufen. Die Zielzellen der Erreger sind in der Regel die Epithelzellen der Konjunktiva sowie der Schleimhäute des Respirationstraktes und des Genitaltraktes.

Das Erregerreservoir für *Chlamydia trachomatis* und *Chlamydia pneumoniae* (neue Nomenklatur auch: *Chlamydophila pneumoniae*) ist der Mensch, während für *Chlamydia psittaci* (neue Nomenklatur auch: *Chlamydophila psittaci*) vor allem Vögel aber auch Nutztiere wichtige Infektionsquellen sein können. Aufgrund ihrer intrazellulären Lage haben Chlamydien Strategien entwickelt, sich der Wirtsabwehr zu entziehen, so dass eine Chronifizierung der Infektion mit der Gefahr von Folgeerkrankungen nicht selten ist. In solchen Fällen ist die Diagnose durch den Erregernachweis meist nicht mehr möglich, wohingegen der Nachweis speziesspezifischer Antikörper erhebliche diagnostische Bedeutung erlangt.

Chlamydien können sich auch in Zellen der Wirtsabwehr wie Monozyten und Lymphozyten vermehren. Möglicherweise gelingt es den Erregern dadurch vom Ort der Primärinfektion zu disseminieren und somit in andere Kompartimente (Gelenke, Gefäße) zu gelangen. Die Primärinfektion verläuft klinisch oft uncharakteristisch und oligosymptomatisch, wodurch die Ausbreitung und die Weiterverbreitung der Infektion begünstigt wird. Da eine Primärinfektion offenbar keine vollständige Immunität induziert, sind Reinfektionen gerade bei *Chlamydia pneumoniae* und *Chlamydia trachomatis* nicht selten. Dementsprechend ist die Therapie oft langwierig und gelingt nur mit Antibiotika, die intrazellulär hohe Wirkstoffspiegel erreichen. Gerade wegen ihrer uncharakteristischen klinischen Symptomatik spielt für den Nachweis von Chlamydien-Infektionen die Labordiagnostik eine entscheidende Rolle.

*Chlamydia trachomatis* gehört in den Industrienationen zu den häufigsten Ursachen von sexuell übertragbaren Erkrankungen (STD). Nach Schätzungen des US-amerikanischen "Center of Disease Control and Prevention" sind mehr als 700 Millionen Menschen mit *Chlamydia trachomatis* infiziert. Die jährliche Inzidenz liegt bei 50 Millionen. Deutschlandweit wird mit einer jährlichen Inzidenz von 300.000 gerechnet (insgesamt ca. 1,15 Millionen). In den Entwicklungsländern verursacht der Erreger mit dem Trachom eine chronische Keratokonjunktivitis, die nach wie vor in diesen Ländern die häufigste Ursache der vermeidbaren Erblindung ist.

Das Erkrankungsspektrum von *Chlamydia trachomatis* ist abhängig vom Serotyp des Erregers. Die Serotypen A-C verursachen das Trachom, die Serotypen L1-L3 verursachen das Lymphogranuloma venereum, eine invasiv in Stadien verlaufende sexuell übertragbare Erkrankung mit vorwiegendem Vorkommen in den Tropen. Die Serotypen D-K dagegen spielen in den Industrienationen die entscheidende Rolle als Ursache von urogenitalen Infektionen, welche zur infektionsbedingten Sterilität und postinfektiösen Arthritis führen können. Die perinatale Übertragung des Erregers von der infizierten Mutter auf das Neugeborene führt zur sogenannten Neugeborenkonjunktivitis oder auch zur Neugeborenenpneumonie. Aufgrund der schweren Folgeerkrankungen, die unerkannte und damit unbehandelte *Chlamydia trachomatis*-Infektionen verursachen können, werden in skandinavischen und angloamerikanischen Ländern Screeninguntersuchungen für Risikogruppen angeboten. Ob in Deutschland auch Screeninguntersuchungen eingeführt werden, ist derzeit noch unklar.

*Chlamydia pneumoniae* ist ein erst seit ca. 15 Jahren bekannter Erreger, der typischerweise Infektionen des Respirationstraktes wie Sinusitis, Pharyngitis, Bronchitis und Pneumoniae hervorrufen kann. Seroepidemiologische Studien weisen darauf hin, dass *Chlamydia pneumoniae* bei Antikörperprävalenzen von über 50% weit verbreitet ist.

Letztlich ist jedoch die klinische Relevanz von *Chlamydia pneumoniae* nicht abschließend geklärt. Im Rahmen des BMBF-Kompetenznetzwerkes "Ambulant erworbene Pneumonie" (CAPNETZ) wird derzeit versucht die Bedeutung des Erregers bei der ambulant erworbenen Pneumoniae zu klären, was sich allerdings durch methodische Schwierigkeiten in der Labordiagnostik insbesondere in der Serologie als extrem schwierig herausstellt. Noch schwieriger jedoch ist es, die Relevanz des Erregers bei extrapulmonalen Erkrankungen abzuschätzen. Von zentraler nicht zuletzt auch gesundheitspolitischer und volkswirtschaftlicher Bedeutung ist die Frage, ob *Chlamydia pneumoniae* an der Atherogenese beteiligt und damit mitverantwortlich ist für die Volkskrankheiten Herzinfarkt und Schlaganfall.

*Chlamydia psittaci* ist der Erreger der Ornithose oder auch der Papageienkrankheit. Es handelt sich dabei um eine Zooanthroponose, die in der Regel von infizierten Zier- oder Nutzvögeln durch Ausscheidung erregerhaltiger Sekrete und Exkremente, aerogen oder seltener durch direkten Kontakt, auf den Menschen übertragen wird. Infektionsgefährdet sind neben den Haltern von Zier- und Nutzvögeln daher vor allem Tierhändler und in der geflügelverarbeitenden Industrie Beschäftigte. Insgesamt ist die Erkrankung in Deutschland selten geworden, allerdings ist aufgrund der schwierigen Diagnostik von einer relativ hohen Dunkelziffer auszugehen.

Hauptmanifestationsform der Infektion ist eine atypische Pneumonie, die mit einer schweren systemischen Symptomatik einhergehen kann. Unbehandelt kann die Infektion tödlich verlaufen. Eine Übertragung von Mensch zu Mensch wurde bisher nicht beobachtet, der Erreger gilt jedoch als hoch kontagiös und wird nach Biostoffverordnung in die Laborsicherheitsstufe 3 eingestuft.

Daneben hat *Chlamydia psittaci* eine große ökonomische Bedeutung im Bereich der Nutztierhaltung, wo schwere systemisch verlaufende Infektionen auftreten können und sich bei infizierten Beständen insbesondere bei Schafen aber auch anderen Nutztieren hohe Abortraten finden.

Die Diagnostik von Chlamydien-Infektionen ist generell schwierig, aufwändig und teuer. Für den Nachweis von chronischen *Chlamydia trachomatis*-Infektionen stehen bisher keine ausreichend empfindlichen, standardisierten und gut evaluierbaren Nachweisverfahren zur Verfügung, die zudem keine falsch-positiven Ergebnisse erzeugen.

Der Nachweis einer *Chlamydia trachomatis-Infektion* wurde bislang beispielsweise mit Nukleinsäure-Amplifikationstests (NAAT, nucleic acid amplification test) durchgeführt. Diese Verfahren wurden zum Nachweis von genitalen Infektionen routinemäßig in klinischen Labors durchgeführt. Derartige, kommerziell erhältliche Assays sind jedoch sehr teuer und zu komplex, um sie im großen Maßstab einzusetzen. Gerade aufgrund der hohen Selektivität dieses Testverfahrens besteht die Gefahr von Kreuzkontaminierungen bzw. falschen Testergebnissen aufgrund falscher Handhabung der Proben. Daher erfordert dieses Testverfahren eine große Aufmerksamkeit bezüglich der Qualitätskontrolle und der Schulung des Laborpersonals.

Des Weiteren besteht die Gefahr, dass sich der Erreger bzw. dessen Nukleinsäuresequenz ändert und ein Nachweis mit den bisherigen Sonden nicht mehr möglich ist. Zudem besteht die Gefahr, dass falsch-positive Ergebnisse erzeugt werden, wenn mehr als eine Chlamydia-Spezies die Nukleinsäuresequenz der Sonde aufweist.

Ein anderes bekanntes Verfahren beruht auf dem Nachweis von Serumantikörpern gegen *Chlamydia trachomatis.* Die bisher auf dem Markt befindlichen gattungsspezifischen Antikörpemachweise erfassen Antikörper gegen alle Chlamydienarten und sind angesichts der hohen Prävalenz von *Chlamydia pneumoniae*-Antikörper positiven Patienten oft sehr schwer zu interpretieren. Speziesspezifische Tests sind technisch sehr aufwändig und schwierig zu beurteilen (Mikroimmunfluoreszenztest).

In der Vergangenheit gab es bereits viele erfolglose Versuche immundominante und spezifische Chlamydien-Antigene zu identifizieren. Insbesondere hat sich die speziesspezifische Diagnostik mittels *Chlamydia pneumoniae-*Antigenen als schwierig erwiesen. Hierbei ergaben sich Diskrepanzen in der Reaktivität zwischen dem bisher geltenden Goldstandard MIF (Mikroimmunfluoreszenztest) und den rekombinanten Antigenen (Maile et al., 2005, "recomLine Chlamydia: a new serological test system for the detection of antibodies against Chlamydia trachomatis, Chlamydia pneumoniae and Chlamydia psittaci", 3. Deutscher Chlamydienworkshop, 09.03.-11.03.2005, Jena).

Die WO 2004/074318 beschreibt Polypeptide von *Chlamydia trachomatis, Chlamydia psittaci* und *Chlamydia pneumoniae* und deren Verwendung in der Diagnostik. Unter mehr als 10 verschiedenen Polypeptiden, die für die Diagnose von *Chlamydia trachomatis* eingesetzt werden sollen, wird unter anderem auch das Protein CT664 erwähnt.

Sharma et al., Infection and Immunity (März 2006), S. 1490-1499 beschreiben die Expression von 156 *Chlamydia trachomatis* Fusionsproteinen, die in Mikrotiterplatten zur Diagnose von Urogenitalinfekten mit *C*. *trachomatis* eingesetzt wurden. In dieser Veröffentlichung wird zwar das Protein CT431 in einer Liste aus 156 Proteinen erwähnt, jedoch wies dieses Antigen keine signifikante Reaktivität mit den untersuchten Seren auf. Diese Literaturstelle bevorzugt den Einsatz anderer Antigene für die Diagnose.

Sanchez-Campillo, Electrophoresis (1999), S. 2269-2279 beschreibt die Identifizierung von immunreaktiven Proteinen von *Chlamydia trachomatis* durch Western Blot Analyse und zweidimensionale Gelelektrophorese mit Patientenseren. Für die Routinediagnostik ist die dort beschriebene Methode ungeeignet, weil zu aufwendig und zu teuer.

Biendo et al., Clinical and Diagnostic Laboratory Immunology (November 1996), S. 706-709 beschreiben die Grenzen des Mikroimmunfluoreszenztests und die Vorteile des Immunblottens bei der Diagnose von Chlamydia-Infektionen.

Die nicht vorveröffentlichte WO 2007/110700 offenbart immunogene Zusammensetzungen, die vor allem als Impfstoffe Verwendung finden sollen. In dieser Veröffentlichung werden Antigene mit den Nummern 456 und 017 erwähnt.

Anfängliche Versuche im Rahmen der vorliegenden Erfindung zur Klonierung und rekombinanten Herstellung von bekannten Chlamydien-Proteinen, wie beispielsweise den Proteinen PGP3 (Maile et al., 2004, "Evaluation of selected recombinant chlamydial antigens for serological diagnosis of Chlamydia trachomatis and Chlamydia pneumoniae infection", 5th Meeting of the European Society for Chlamydia Research, 01-04.09.2004, Budapest), MOMP, OMP2, hsp60, MIP und MOMPIV von *Chlamydia trachomatis* waren für die Entwicklung eines spezifischen Nachweisverfahrens für *Chlamydia trachomatis-*Infektionen nicht geeignet.

Dies zeigt, dass mit Ganzzell-Lysat-Antigenen auf der Basis von gereinigten Chlamydien-Elementarkörperchen keine wesentlichen Fortschritte in der Chlamydien-Serodiagnostik zu erreichen sind. So sind bisher auch keine spezifischen Antigene von *Chlamydia pneumoniae* bekannt, die eine sichere serologische Differenzierung von *Chlamydia pneumoniae* und *Chlamydia trachomatis*-Infektionen zulassen.

Darüber hinaus wurde eine große Anzahl PCR-basierter Verfahren entwickelt, wobei beispielsweise unterschiedliche Zielgene von *Chlamydia pneumoniae* aus respiratorischen und nicht-respiratorischen Proben entwickelt wurden. Viele dieser PCR-Verfahren waren jedoch nicht verlässlich oder stabil genug, um reproduzierbare Ergebnisse in klinischen Routineuntersuchungen zu ermöglichen. Daher wurden diese Assays eher als Forschungswerkzeuge verwendet.

Des Weiteren wurden Enzymimmunoassay-basierende Verfahren zur Detektion von *Chlamydia trachomatis*-Infektionen mittels monoklonaler oder polyklonaler Antikörper verwendet. Diese Tests haben sich bislang jedoch nicht als erfolgreich dargestellt, insbesondere aufgrund ihrer geringen Sensitivität und der von ihnen erzeugten falsch-positiven Testergebnisse.

Eine Aufgabe der vorliegenden Erfindung besteht daher darin, ein selektives Nachweisverfahren für *Chlamydia trachomatis*-Infektionen zur Verfügung zu stellen. Dieses Verfahren soll keine Kreuzreaktivität bezüglich anderer Erreger aufweisen und dadurch insbesondere eine Abgrenzung gegenüber *Chlamydia pneumoniae*-Infektionen ermöglichen.

Eine weitere Aufgabe der vorliegenden Erfindung besteht darin, in der Diagnose festzustellen, um welche Art von Chlamydien-Infektion es sich handelt. In Frage kommt dabei eine akute Infektion mit *Chlamydia pneumoniae* oder eine chronische Infektion mit *Chlamydia trachomatis* sowie eine akute *C*. *trachomatis*-Infektion. Eine weitere Aufgabe der vorliegenden Erfindung besteht in der Bereitstellung von hochspezifischen und/oder hochsensitiven Antigenen zur Differentialdiagnostik von Chlamydia-Infektionen.

Insbesondere muss das Testverfahren eine hohe Sensitivität aufweisen, um den Nachweis chronischer *Chlamydia trachomatis*-Infektionen zu ermöglichen. Des Weiteren soll das Testverfahren zum selektiven Nachweis von *Chlamydia trachomatis*-Infektionen für routinemäßige Laboruntersuchungen aufgrund kostengünstiger, einfacher und sicherer Handhabung geeignet und zusätzlich nicht nur zum Nachweis des Krankheitserregers, sondern auch zur Beurteilung des Fortschritts der Krankheit bzw. des Krankheitsstadiums geeignet sein.

Das selektive Testverfahren soll insbesondere für die Anwendung in Multiparameter-Tests auf der Basis von Biochip- oder Luminex-Technologie geeignet sein, insbesondere, da Chlamydien-Infektionen auch Auslöser von Krankheitsbildern mit typischen Folgekomplikationen wie der reaktiven Arthritis sein können. Gerade die Arthritisdiagnostik ist gegenwärtig durch die Notwendigkeit unter vielen potentiellen Erregern die genaue Ursache zu finden, mit einem sehr kostenintensiven Aufwand von Einzeltests verbunden.

Eine Aufgabe ist es auch, ein Testverfahren für den veterinärmedizinischen Bereich zur Verfügung zu stellen, da auch hier ein großer Bedarf nach selektiven Tests besteht.

Die erfindungsgemäße Aufgabe wurde durch den Einsatz von individuell immobilisierten rekombinanten Antigenen anstelle von herkömmlich verwendeten Lysaten und Elementarkörperchen gelöst und stellt eine wesentliche Verbesserung im Bereich der Chlamydien-Serodiagnostik dar. Die Identifizierung der für *Chlamydia trachomatis* spezifischen Antigene ist in den Beispielen dargestellt.

Die erfolgreiche Charakterisierung immundominanter und spezies-spezifischer Chlamydien-Proteine, die als Antigene für die Diagnostik eingesetzt werden können, konnte unerwarteterweise durch ausschließliche in-vivo-Expression erreicht werden, wobei Antigene identifiziert wurden, die konventionellen diagnostischen Ansätzen entgehen. Hierbei wurden alle Antigene mittels 2D-Immunelektophorese und Western-Blots mit Hilfe charakterisierter Patienten-Seren sowie konsekutiver massenspektrometrischer Analyse reaktiver Spots identifiziert. Dies ist ebenfalls in den Beispielen beschrieben.

Die vorteilhaft hohe Seroreaktivität der erfindungsgemäßen Antigene wurde dadurch erreicht, dass die Kandidaten-Antigene in bakteriellen Expressionsvektoren kloniert und rekombinant exprimiert wurden und nach einer eventuellen Expressionsoptimierung und Proteinreinigung in Line-Assays analysiert wurden, um eine optimale Kombination an rekombinanten Antigenen zu finden. Bei den "Line-Assays" werden mehrere meist rekombinant hergestellte Antigene als schmale Banden auf einen geeigneten Träger (bspw. Nitrocellulose) aufgebracht und die Teststreifen mit der Untersuchungsflüssigkeit (bspw. Serum) umgesetzt. Diese Antigene ermöglichen auf vorteilhafte Weise einerseits eine genaue Speziesdifferenzierung und andererseits eine Unterscheidung zwischen aktiven und abgelaufenen Chlamydien-Infektionen. Dies war bislang nur mit PCR-Verfahren möglich.

Der stadienabhängigen, spezies-spezifischen Serologie zur Diagnose von *Chlamydia* trachomatis-Infektionen kommt eine wichtige Rolle bei therapeutischen Entscheidungen aber auch bei der Bewertung der Prognose bestehender Krankheitszustände zu.

Wie bereits erwähnt, ermöglicht die Verwendung der erfindungsgemäßen Antigene in einem Multiparameter-Verfahren auf vorteilhafte Weise den gleichzeitigen Nachweis verschiedener Erreger, insbesondere den Nachweis der verschiedenen Chlamydia-Erreger, *Chlamydia trachomatis, Chlamydia pneumoniae* und *Chlamydia psittaci.* Ein Vorteil der Verwendung von mehr als einem Antigen im Nachweisverfahren liegt darin, dass die Testsicherheit erhöht wird. Die erfindungsgemäßen Antigene besitzen einerseits eine höhere Spezifität bei klinisch relevanten Infektionen mit *Chlamydia trachomatis,* sowie andererseits eine geringere Kreuzreaktivität mit *Chlamydia pneumoniae* als bislang bekannte rekombinant hergestellte Antigene (siehe Tabelle 3). Die erfindungsgemäßen Antigene waren bislang nicht im Zusammenhang mit dem selektiven Nachweis von *Chlamydia trachomatis* bekannt.

Das Antigen CT456-TARP wurde zwar bereits beschrieben (Clifton et al., Proc Natl Acad Sci USA. 2004, Jul 6;101(27):10166-71; Clifton et al., Infect Immun. 2005, Jul;73(7):3860-8; Jewett et al., Proc Natl Acad Sci U S A. 2006 Oct 17;103(42):15599-604.), jedoch nicht im Zusammenhang mit dem Nachweis von *Chlamydia trachomatis.* Es wurde im Rahmen der vorliegenden Erfindung festgestellt, dass es sich hierbei um ein hochspezifisches und hochsensitives Antigen handelt, das für die Differentialdiagnostik von Chlamydia-Infektionen besonders geeignet ist.

Einige weitere immunreaktive Antigene von *Chlamydia trachomatis* wurden bereits beschrieben: CT858-CPAF (Sharma et al., Infect Immun. 72(12):7164-71); CT089 und CT795 (Sharma et al., Infect Immun. 74(3):1490-99) und CT813 (Chen et al., Infect Immun. 74(8):4826-40). Diese vier Proteine wurden zu Vergleichszwecken im Rahmen der vorliegenden Anmeldung untersucht (Tabelle 3). Die Ergebnisse zeigen, dass die vier bekannten Antigene CT858-CPAF, CT089, CT795 und CT813 bei Infektionen mit *Chlamydia pneumoniae* sowie bei gesunden Blutspendern mit Antikörpern gegen *Chlamydia trachomatis* häufiger als die erfindungsgemäßen Antigene reagieren (siehe Tabelle 3).

Die vorliegende Erfindung betrifft daher ein Verfahren zum selektiven Nachweis von *Chlamydia trachomatis -* Infektionen, wobei die Antigene ausgewählt werden aus der Gruppe bestehend aus: CT017, CT098, CT318-L1P, CT431, CT456-TARP, CT603-TSAP und CT664, sowie zusätzlich bevorzugt auch aus Fragmenten und Teilsequenzen der vorstehenden Antigene oder aus im Wesentlichen dazu gleiche Antigenen, die dazu verwendet werden, Antikörper in Proben zu detektieren. Es werden mindestens drei, bevorzugt mindestens vier, weiter bevorzugt mindestens fünf und am meisten bevorzugt mindestens sechs Antigene aus der Gruppe von Antigenen bestehend aus: CT017, CT098, CT318-L1P, CT431, CT603-TSAP und CT664 ausgewählt.

In einer bevorzugten Ausführungsform der Erfindung zum Nachweis von chronischen Erkrankungen sind die Antigene ausgewählt aus der Gruppe bestehend aus: CT017, CT098, CT318-L1P, CT431, CT603-TSAP und CT664. Bevorzugt für den Nachweis von chronischen Erkrankungen bzw. Infektionen sind die Antigene aus der Gruppe ausgewählt bestehend aus: CT017, CT098, CT318-L1P und CT664, besonders bevorzugt sind die Antigene: CT603-TSAP und CT664.

Bei dem erfindungsgemäßen Verfahren sowie in den Testkits oder Biochips können entweder die kompletten oder nahezu kompletten Polypeptide eingesetzt werden. Es ist aber auch möglich, Fragmente hiervon in den Tests einzusetzen. Für die Feststellung, welche Fragmente für die immunologischen Tests besonders geeignet sind, wird zunächst aufgrund der dreidimensionalen Struktur des Polypeptids bzw. aufgrund von Hydrophilie-/Hydrophobie-Blots festgestellt, welche Bereiche des Polypeptids sich an der Oberfläche und damit in Kontakt mit dem Immunsystem des Wirtsorganismus befinden. Diese Fragmente werden dann in Abschnitten von wenigstens 15, bevorzugt wenigstens 20, noch bevorzugter wenigstens 30 und besonders bevorzugt wenigstens 50 Aminosäuren hergestellt. Die Herstellung kann entweder durch chemische Synthese oder rekombinant durch Expression, beispielsweise als Fusionspolypeptid mit einem immunologisch inaktiven Trägerprotein erfolgen. Diese Fragmente werden dann in geeigneten Tests, beispielsweise sogenannten Line Assays, ELISA Tests oder Western Blots mit verschiedenen, möglichst genau charakterisierten Seren umgesetzt, von denen bekannt ist, welche Infektion vorliegt. Hierbei kommt es auf die genaue Bestimmung des Erregers sowie den aktuellen Zustand der Infektion (akut, lang zurückliegend oder chronisch) an. Aus den Testergebnissen kann dann geschlossen werden, welche dieser Fragmente für den Einsatz in den diagnostischen Testverfahren geeignet, gut geeignet, besonders geeignet oder nicht geeignet sind.

Eine weitere Ausführungsform der Erfindung betrifft ein Verfahren, das dadurch gekennzeichnet ist, dass die Detektion der Antikörper den Nachweis eines Antigen-/ Antikörperkomplexes umfasst. Eine bevorzugte Ausführungsform der Erfindung betrifft ein in vitro Verfahren zur Analyse von Proben von menschlichen Patienten. In einer bevorzugten Ausführungsform des Verfahrens werden die zu untersuchenden Proben einer Zelllyse unterzogen, bevor sie mit den Antigenen in Kontakt gebracht werden. Hierdurch werden Erreger, beziehungsweise erregerspezifische Antigene aus den infizierten Zellen freigesetzt

Bei einer weiteren bevorzugten Ausführungsform des Verfahrens erfolgt die Detektion der Antikörper mittels ELISA (Enzyme-Linked Immunosorbent Assay), Westem-Blot oder Line-Assay, besonders bevorzugt ELISA. Besonders bevorzugt ist hierbei die Verwendung von ELISA, Western-Blot und Line-Assay mit peroxidasekonjugierten Sekundärantikörpern, wobei der Nachweis der Antigen-Antikörper-Reaktion über ein Färbesubstrat (TMB, Tetramethylbenzidin) erfolgt.

Eine weiter bevorzugte Ausführungsform des Verfahrens ist dadurch gekennzeichnet, dass bei dem Verfahren zum selektiven Nachweis von *Chlamydia trachomatis* Infektionen die Menge an Antikörpern in der Probe quantitativ bestimmt wird.

In einer bevorzugten Ausführungsform des Verfahrens erfolgt der Nachweis der Antigen-Antikörper-Reaktion unter Verwendung von peroxidasekonjugierten Sekundärantikörpern und dem Färbesubstrat (TMB).

Eine weitere Ausführungsform der Erfindung betrifft ein Verfahren zum Analysieren des Krankheitsverlaufs einer *Chlamydia trachomatis* Infektion, worin in bestimmten Zeitabständen Proben von einem Patienten genommen werden und die Proben nach dem erfindungsgemäßen Verfahren untersucht werden. Bevorzugt ist hierbei, dass bei dem erfindungsgemäßen Verfahren zum selektiven Nachweis von *Chlamydia trachomatis -* Infektionen die Menge an Antikörpern in der Probe quantitativ bestimmt wird.

Des Weiteren betrifft die Erfindung ein Testkit zum selektiven Nachweis von *Chlamydia trachomatis -* Infektionen, dadurch gekennzeichnet, dass mindestens drei Antigene, ausgewählt aus der Gruppe bestehend aus: CT017, CT098, CT318-L1P, CT431, CT456-TARP, CT603-TSAP, und CT664, verwendet werden, um Antikörper in Proben von Patienten zu detektieren. Es werden mindestens drei, weiter bevorzugt mindestens vier, und am meisten bevorzugt mindestens sechs Antigene aus den vorstehend genannten Gruppen von Antigenen ausgewählt. In einer besonders bevorzugten Ausführungsform wird die gesamte Gruppe der *Chlamydia trachomatis -* spezifischen Antigenen für das Nachweisverfahren verwendet.

Zudem betrifft die Erfindung einen Biochip der Antigene, ausgewählt aus der Gruppe bestehend aus: CT017, CT098, CT318-L1P, CT431, CT456-TARP, CT603-TSAP, und CT664, für den Nachweis von *Chlamydia trachomatis* -spezifischen Antikörpern enthält. Es werden mindestens drei, bevorzugt mindestens vier, und am meisten bevorzugt mindestens sechs Antigene aus den vorstehend genannten Gruppen von Antigenen ausgewählt.

Eine Ausführungsform des Biochips zeichnet sich dadurch aus, dass die Antigene, ausgewählt aus der Gruppe bestehend aus: CT017, CT098, CT318-L1P, CT431, CT456-TARP, CT603-TSAP, und CT664, auf einer Stelle eines festen Trägers, beispielsweise aus Glas oder Plastik bzw. einer Membran, bevorzugterweise einer Membran, angebracht sind und Stellen auf dem festen Träger mit andere Antigenen räumlich getrennt davon sind. Weiter bevorzugt ist hierbei, dass die Antigen-/ Antikörperkomplexe durch Farbreaktionen auf dem festen Träger, bevorzugt einer Membran, detektiert bzw. sichtbar gemacht werden können. Bevorzugterweise erfolgt der Nachweis unter Verwendung von Farbreaktionen, bevorzugterweise mit TMB (Tetramethylbenzidin), oder mittels Fluoreszenzfarbstoffen wie bevorzugterweise, Cy3- bzw. Cy5-Farbstoff. Besonders bevorzugt ist hierbei, dass ein Farbstoff entweder an den Antigen-/ Antikörperkomplex bindet, oder in diesen interkaliert. Weiter bevorzugt ist, dass räumlich getrennt von den Stellen auf der Membran mit den erfindungsgemäßen Antigenen weitere Antigene angebracht sind, die einen spezifischen Nachweis gegen *Chlamydia pneumoniae* ermöglichen. Für Biochip-Anwendungen mit festen Biochip-Oberflächen (Kunststoff) werden besonders bevorzugt die Cy3-, Cy5-Farbstoffe verwendet.

Für das erfindungsgemäße Nachweisverfahren werden biologische Proben verwendet. Diese biologischen Proben enthalten die *Chlamydia* trachomatis-spezifischen Antikörper. Die biologische Probe kann von menschlichen oder tierischen Patienten stammen, bevorzugterweise von menschlichen Patienten. Die biologische Probe kann jede Probe sein, die Körperflüssigkeit oder Gewebe, wie beispielsweise Blut oder Knochenmark enthält. Bevorzugterweise wird Serum oder Plasma verwendet. Zusätzlich bevorzugt ist, dass die Proben einer Zelllyse unterzogen werden, bevor sie mit den Antigenen in Kontakt gebracht werden. Verfahren zur Probenbereitung für Immunoassays sind dem Fachmann bekannt. Die Aufbereitung der Proben kann hierbei beispielsweise das Zentrifugieren, Ausfällen, Aufkonzentrieren, Filtrieren, Dialysieren oder Verdünnen der Probe umfassen. Die Art der Probenaufbereitung ist hierbei von der Technik der Detektion der Antikörper abhängig.

Das erfindungsgemäße Verfahren beruht auf üblichen, dem Fachmann bekannten Techniken der Molekularbiologie, Mikrobiologie, rekombinaten DNA, und Immunologie. Diese Techniken sind in der Literatur ausführlich beschrieben. Diesbezüglich wird insbesondere verwiesen auf: Molecular Cloning, A Laboratory Manual, zweite Ausgabe (1989); DNA Cloning, Volumes I und II (D. N. Glover, Ed., 1985); Oligonucleotide Synthesis (M. J. Gait, Ed, 1984); Nucleic Acid Hybridization (B. D. Harnes & S. J. Higgins, Eds., 1984); Transcription And Translation (B. D. Harnes & S. J. Higgins, Eds., 1984); Animal Cell Culture (R. I. Freshney, Ed., 1986); Immobilized Cells And Enzymes (IRL Press, 1986); B. Perbal, A Practical Guide To Molecular Cloning (1984); Die Serien, Methods In Enzymology (Academic Press, Inc.); Gene Transfer Vectors For Mammalian Cells (J. H. Miller und M. P. Calos, Eds., 1987, Cold Spring Harbor Laboratory), Methods in Enzymology Vol. 154 und Vol. 155 (Wu and Grossman, bzw. Wu), Mayer und Walker, Eds. (1987), Immunochemical Methods In Cell And Molecular Biology (Academic Press, London), Scopes, (1987), Protein Purification: Principles And Practice, zweite Ausgabe, (Springer-Verlag, N.Y.), und Handbook Of Experimental Immunology, Volumes I - IV (D. M. Weir und C. C. Blackwell, Eds, 1986).

Der Nachweis einer *Chlamydia trachomatis -* Infektion erfolgt über den Nachweis von *Chlamydia trachomatis* spezifischen Antikörpern in den Proben durch die erfindungsgemäßen Antigene. Der Begriff "Antikörper" bezeichnet ein Immunoglobulin oder ein Derivat davon, das an die erfindungsgemäßen Antigene binden kann.

Die erfindungsgemäßen "Antigene" können an die *Chlamydia trachomatis- spezifischen Antikörper* binden. Die Erfindung betrifft Antigene mit den in den Sequenzprotokollen angegebenen Sequenzen und zusätzlich bevorzugt auch immunogene Fragmente bzw. Teilsequenzen der Aminosäuresequenzen, die in den Sequenzprotokollen angegeben sind und zusätzlich bevorzugt auch immunogene Antigene, die zu den Aminosäuresequenzen aus den Sequenzprotokollen im Wesentlichen gleich sind.

Geeignete Fragmente bzw. Teilsequenzen der Aminosäuresequenzen der Antigene haben üblicherweise eine Länge von mindestens 15 Aminosäuren, bevorzugterweise mindestens 20 Aminosäuren und noch bevorzugter mindestens 25 Aminosäuren. Für die Tests werden solche Fragmente der erfindungsgemäß eingesetzten Antigene verwendet, die wenigstens ein diagnostisch relevantes spezies-spezifisches Epitop aufweisen. Dem Durchschnittsfachmann sind verschiedene Methoden zur Kartierung von Antigenen bekannt. Durch verschiedene Methoden des Epitopmappings kann bestimmt werden, welche Bereiche für die immunologische Diagnostik besonders relevant sind. Lineare Epitope finden sich auf kurzen Aminosäureabschnitten. Konformationsepitope entstehen durch die räumliche Konfiguration des Antigens. Daher sind bevorzugte Fragmente, die Konformationsepitope aufweisen, in der Regel länger, üblicherweise wenigstens 50 Aminosäuren, bevorzugter wenigstens 75 Aminosäuren. Dem Durchschnittsfachmann ist allerdings auch bekannt, dass nur einige wenige Aminosäuren am Epitop beteiligt sind. Andere Aminosäuren, die sich beispielsweise im Inneren des Antigens befinden können verändert sein, ohne dass dies die diagnostische Bedeutung des Epitops verändert.

Der Ausdruck: "Antigene, die zu den Aminosäuresequenzen aus den Sequenzprotokollen im Wesentlichen gleich sind" umfasst hierbei Aminosäuresequenzen bei denen (beispielsweise durch Mutation) 1 bis 20, bevorzugt 1 bis 15, noch bevorzugter, 1 bis 10, besonders bevorzugt 1 bis 5, und am meisten bevorzugt 1 bis 3 Aminosäuren substituiert, deletiert oder addiert wurden im Vergleich zu den Sequenzen aus den Sequenzprotokollen.

Dem Fachmann ist bekannt, dass die antigenen Eigenschaften eines Proteins oder Polypeptids häufig nicht verändert werden, wenn eine oder einige wenige Aminosäuren an immunologisch nicht bedeutenden Stellen verändert werden. Es können daher leicht Aminosäuren verändert werden, wenn sie einen ähnlichen Charakter aufweisen oder die Tertiärstruktur des Proteins nicht oder nicht wesentlich beeinflussen. Häufig beeinflusst beispielsweise ein Austausch Gly zu Ala oder umgekehrt den immunologischen Charakter nicht.

Geeignete Fragmente bzw. Teilsequenzen oder "im Wesentlichen" gleiche Antigene sind hierbei Proteine, die eine Selektivität gegenüber *Chlamydia trachomatis*-spezifischen Antikörpern aufweisen. Bevorzugt kommt es bei Durchführung eines erfindungsgemäßen Nachweisverfahrens gemäß den Beispielen bei Verwendung von positive Blutspenderseren ohne klinischem Verdacht auf eine chronische *C*. *trachomatis*-Infektion bei maximal 3 von 6 Personen, weiter bevorzugt maximal 2 von 6 Personen und noch weiter bevorzugt bei maximal 1 von 6 Personen zu einem falsch-positiven Ergebnis.

Die Antigene werden ausgewählt aus der Gruppe bestehend aus CT017, CT098, CT318-L1P, CT431, CT456-TARP, CT603-TSAP und CT664 oder Fragmenten davon. Weiter bevorzugt sind die Antigene ausgewählt aus der Gruppe bestehend aus: CT017, CT098, CT318-L1 P, CT431, CT603-TSAP und CT664.

In einer bevorzugten Ausführungsform zum Nachweis von chronischen Erkrankungen sind die Antigene besonders bevorzugt ausgewählt aus der Gruppe bestehend aus: CT017, CT098, CT318-L1P, CT431, CT603-TSAP und CT664. Noch weiter bevorzugt für den Nachweis von chronischen Erkrankungen bzw. Infektionen sind die Antigene aus der Gruppe bestehend aus: CT017, CT098, CT318-L1P und CT664. Am meisten bevorzugt sind die Antigene: CT603-TSAP und CT664.

Tabelle 1 zeigt die erfindungsgemäßen Antigene mit ihren Sequenznummern (SEQ ID NO), wobei die Sequenzen im Sequenzprotokoll wiedergegeben sind.

Es werden mindestens drei, bevorzugt mindestens vier, und weiter bevorzugt mindestens sechs Antigene aus den vorstehend genannten Gruppen von Antigenen ausgewählt. In einer besonders bevorzugten Ausführungsform wird die gesamte Gruppe der *Chlamydia trachomatis -* spezifischen Antigenen für das Nachweisverfahren verwendet.

zusätzlich zu den oben angegebenen Antigenen können auch weitere *Chlamydia trachomatis -* spezifische Antigene in dem erfindungsgemäßen Nachweisverfahren verwendet werden. Falls die Detektion der Antigen-/Antikörperkomplexe selektiv erfolgt, können zusätzlich und gleichzeitig auch andere Antigene gegen andere Erreger in dem gleichen Nachweisverfahren verwendet werden. Beispielsweise könnten die Antigen-/Antikörperkomplexe der verschiedenen Erreger aufgrund unterschiedlicher spektroskopischer Eigenschaften nebeneinander analysiert werden.

**Tabelle 1. Chlamydia trachomatis-Antigene (Bei den Genbanknummern handelt es sich um die Nummern, unter denen die Gesamtgenome zweier Isolate von Chlamydia trachomatis zu finden sind. Einen separaten Eintrag für die einzelnen Gene gibt es bisher nicht. Diese sind anhand der Nummer des offenen Leserahmens mit vorgestelltem CT für Chlamydia trachomatis im Gesamtgenom unterschieden; Maßgeblich sind jedoch die Sequenzprotokolle):**

| Antigen | SEQ ID NO | Genbank-Nummer |
|---|---|---|
| CT017 | 1 | NC000117 |
| CT098 | 2 | NC000117 |
| CT318-L1P | 3 | CP000051 |
| CT431 | 4 | NC000117 |
| CT456-TARP | 5 | NC000117 |
| C/603-TSAP | 6 | NC000117 |
| CT664 | 7 | NC000117 |

Die Herstellung der Antigene kann durch bekannte Verfahren erfolgen. Insbesondere können die Antigene in bakteriellen Expressionsvektoren kloniert und rekombinant exprimiert werden. Hierzu geeignete Organismen sind dem Fachmann bekannt und umfassen beispielsweise das Bakterium *Escherichia coli.* Die Herstellung der Antigene ist in Beispiel 1 beschrieben.

Der Nachweis der Antikörper kann bevorzugt durch Immunoassays erfolgen. Hierbei können entweder direkte oder indirekte Immunoassays verwendet werden. Diese Assays umfassen, sind jedoch nicht darauf beschränkt: kompetitive Bindungsassays, nicht-kompetitive Bindungsassays, Radioimmunoassays, Enzyme-Linked Immunosorbent-Assays (ELISA), Western-Blot, Line-Assay, Sandwich-Assays, Fällungsreaktionen, Gel-Diffusion-Immunodiffusionsassays, Agglutinationsassays, Fluoreszenzimmunoassays, Chemolumineszenzimmunoassays, ImmunoPCR-Immunoassays, Protein A oder Protein G Immunoassays und Immunoelektrophoreseassays.

Besonders vorteilhaft ist die Verwendung eines Enzyme-Linked Immunosorbent Assays (ELISA), Western-Blots oder Line-Assays, weiter bevorzugt ELISA. Diese Verfahren basieren auf Antigen-Antikörper-Wechselwirkungen, wobei der entstehende Antigen-/Antikörperkomplex durch bekannte Nachweisverfahren detektiert werden kann. Besonders bevorzugt ist hierbei die Verwendung von ELISA-Verfahren, Western-Blots und Line-Assays mit peroxidasekonjugierten Sekundärantikörpern, wobei der Nachweis der Antigen-Antikörper-Reaktion über ein Färbesubstrat (TMB) erfolgt.

Insbesondere geeignet sind ELISA-Testverfahren, in denen die Antigene an eine feste Phase meist nicht-kovalent (hydrophobe Wechselwirkungen) gebunden sind und mit einer Probenflüssigkeit in Kontakt gebracht werden. Als feste Phase kann beispielsweise eine Mikrotiterplatte verwendet werden. Das Protein (Antigen) kann dann mit der festen Phase bzw. der Oberfläche der Mikrotiterplatte, üblicherweise aus Polystyrol, z.B. bei hohem pH-Wert wechselwirken. Bevorzugterweise wird ein Biochip als feste Phase verwendet.

Indikatorsysteme für den Nachweis der Antigen-/Antikörperkomplexe sind dem Fachmann bekannt. Beispielsweise kann nach Bildung des Antigen-/Antikörperkomplexes ein zweiter Antikörper verwendet werden, der den konstanten Teil des ersten Antikörpers erkennt oder auf andere Weise spezifisch an den Antigen-/Antikörperkomplex bindet. Der zweite Antikörper bzw. die Verbindungen zur Detektion des Antigen-/Antikörperkomplexes, beispielsweise ein monoklonaler oder polyklonaler Antikörper oder ein Fragment davon, besitzt bevorzugterweise eine Markierung. Noch bevorzugter weist er eine nicht-radioaktive Markierung wie beispielsweise eine Enzym-Markierung, Fluoreszenz-Markierung, Lichtemissions-Markierung und so weiter auf. Besonders bevorzugt weist er eine Enzym-Markierung wie eine alkalische Phosphatase, β-Galactosidase oder Meerrettichperoxidase auf.

Der zweite Antikörper kann beispielsweise an eine alkalische Phosphatase gekoppelt sein, wobei die alkalische Phosphatase dann eine enzymatische Chromogenumwandlung katalysiert. Das farblose Chromogen wird dann zu einem Farbstoff umgesetzt und vermessen. Die Menge an freigesetztem Farbstoff korreliert dann mit der Menge des gesuchten Antikörpers. Weitere kompetitive oder nicht-kompetitive Messverfahren sind dem Fachmann bekannt.

Des Weiteren können auch Verbindungen oder Marker zur direkten Detektion des Antigen-/Antikörperkomplexes verwendet werden, so wie beispielsweise radioaktive, fluoreszierende biologische oder enzymatische Tags. Besonders vorteilhaft ist es, wenn der Antigen-/Antikörperkomplex mit dem Farbstoff verbunden ist und die Stelle auf der Membran detektiert werden kann, auf der der Antigen-/Antikörperkomplex mit den erfindungsgemäßen Antigenen aufgebracht ist. Dies ermöglicht den selektiven, gleichzeitigen Nachweis von verschiedenen Krankheitserregern, beispielsweise *Chlamydia trachomatis oder Chlamydia pneumoniae.* Bevorzugt können dann die Testergebnisse optisch ausgewertet werden, wobei beispielsweise farbige Stellen den positiven Nachweis der Krankheitserreger anzeigen, deren Antigene an die Stelle angebracht wurden. Des Weiteren ist bevorzugt, dass sich die optisch detektierbaren Antigen-/Antikörperkomplexe farblich bzw. in ihrem Wellenlängenabsorptionsverhalten unterscheiden. So können die Antigen-/Antikörperkomplexe zusätzlich zu oder anstelle von einer räumlichen Kennzeichnung noch durch ihr Absorptionsverhalten charakterisiert werden. Die Verwendung von peroxidasekonjugierten Sekundärantikörpern, wobei der Nachweis der Antigen-Antikörper-Reaktion über ein Färbesubstrat (TMB) erfolgt, bzw. die Verwendung von Fluoreszenzfarbstoffen wie Cy3 bzw. Cy5 ist bevorzugt. Alle Verfahren zur Detektion des Antigen-/Antikörperkomplexes können sowohl für die Verfahren mit als auch ohne Biochip bzw. festen Träger eingesetzt werden.

Eine weitere Ausführungsform der Erfindung umfasst ein Testkit zum selektiven Nachweis von *Chlamydia trachomatis*-Infektionen. Das Testkit umfasst die oben angegebenen *Chlamydia trachomatis*-spezifischen Antigene. Die an anderer Stelle beschriebene Anzahl an verschiedenen *Chlamydia trachomatis*-spezifischen Antigenen ist ebenfalls für das Testkit geeignet. Die Durchführung des Verfahrens und die Techniken zur Detektion der Antigen-/Antikörperkomplexe sind auf das Testkit anwendbar. Insbesondere kann das Testkit zusätzlich die zur Detektion der Antigen-/Antikörperkomplexe notwendigen Substanzen enthalten.

Des Weiteren kann das Testkit zusätzlich andere Komponenten wie Waschpuffer oder Zusammensetzungen, die einen *Chlamydia trachomatis*-spezifischen Antikörper als Standard enthalten, umfassen. Zusätzlich kann das Testkit auch eine Mikrotiterplatte oder einen Biochip umfassen, auf dem die Antikörper immobilisiert sind. Des Weiteren kann das Testkit auch Informationsmaterialien wie beispielsweise eine Gebrauchsanweisung enthalten, die die Probenbereitung und/oder die Vorschrift zur Durchführung des Assays beschreiben.

Eine Ausführungsform der Erfindung umfasst einen Biochip, auf dem die erfindungsgemäßen Antigene aufgebracht sind. Die an anderer Stelle beschriebene Anzahl an verschiedenen *Chlamydia trachomatis*-spezifischen Antigenen ist ebenfalls für das Verfahren mit einem Biochip geeignet. Die Durchführung des Verfahrens und die Techniken zur Detektion der Antigen-/Antikörperkomplexe sind auch auf das Verfahren mit einem Biochip anwendbar. Insbesondere können die Antigene auf der Membran des Biochips angebracht werden.

Für die Membran können beispielsweise poröse, natürlich vorkommende oder synthetische Polymere, Glas, keramische Materialien, Zellulosematerialien oder Ähnliches verwendet werden. Die Materialien könne beispielsweise aus Filmen, Fasern oder Partikeln (beispielsweise Kügelchen, die durch Kleb- oder Bindematerialien zusammengehalten werden) zu einer Membran gefertigt werden. Besonders bevorzugt besteht die Membran aus Polycarbonat, Nitrozellulose, Zelluloseacetat, Polyamid, Polyester, Polyvinylidenfluorid oder Nylon. Am meisten bevorzugt besteht die Membran aus Nitrozellulose. Der Ausdruck "Nitrozellulose" bezieht sich auf Esterzellulose mit Nitrat-Gruppierungen. Der Ausdruck "Nitrozellulose" umfasst zudem Esterzellulose mit Nitrat-Gruppierungen alleine oder mit einem Gemisch aus verschiedenen Estern, die durch andere Säuren als Nitriersäure erzeugt wurden, insbesondere aliphatische carbozyklische Säuren. Der Ausdruck "aufgebracht" bedeutet, dass die Antigene eine ausreichende Affinität zu der Membran aufweisen oder sogar chemisch mit dieser verbunden sind, so dass das Aufbringen der Probenflüssigkeit bzw. der eventuell weiter notwendigen Wasch- und Reaktionslösungen nicht zur Ablösung dieser Antigene führt.

Das erfindungsgemäße Verfahren kann wie folgt durchgeführt werden (siehe auch Beispiele):
Der erste Schritt der serologischen Nachweisreaktion ist die Immobilisierung der Antigene auf dem festen Träger bzw. der festen Phase, beispielsweise der Nitrozellulosemembran, der Mikrotiterplatte oder dem Biochip. Hierbei werden die erfindungsgemäßen Antigene oder eine Auswahl aus der Gruppe der erfindungsgemäßen Antigene, beispielsweise durch hydrophobe Wechselwirkungen an der Oberfläche immobilisiert.

Nach einem eventuellen Waschschritt werden die immobilisierten Antigene dann mit der Probenflüssigkeit in Kontakt gebracht. Die Aufarbeitung der Proben kann nach den oben beschriebenen Verfahren und wie in den Beispielen erläutert erfolgen, ist jedoch nicht darauf beschränkt. Durch die Antigen-/Antikörper-Wechselwirkung werden die *Chlamydia* trachomatis-spezifischen Antikörper aus der Probenflüssigkeit an die erfindungsgemäßen Antigene gebunden. Es bilden sich Antigen-/Antikörperkomplexe.

Nach einer geeigneten Inkubationszeit wird der feste Träger gewaschen und von den Rückständen der Probe befreit. Anschließend wird eine Lösung zum Detektieren des Antigen-/Antikörperkomplexes mit den Antigen-/Antikörperkomplexen in Kontakt gebracht, wobei eine quantitative oder qualitative Detektion beispielsweise spektroskopisch erfolgen kann. Hierbei kann beispielsweise eine automatisierte Messung der Farbtiefe des Chromogens durchgeführt werden.

In einer Ausführungsform der Erfindung wird der Krankheitsverlauf der *Chlamydia trachomatis*-Infektion ausgewertet. Für diese Ausführungsform des Verfahrens kann auch das erfindungsgemäße Testkit oder der erfindungsgemäße Biochip verwendet werden. Hierbei werden mindestens zwei, weiter bevorzugt mindestens drei, noch weiter bevorzugt mindestens vier und am meisten bevorzugt mindestens fünf Proben des gleichen Patienten zu unterschiedlichen Zeitpunkten getestet und die Anwesenheit von *Chlamydia trachomatis* bzw. die Menge der *Chlamydia trachomatis*-spezifischen Antikörper gemessen. Dieses Verfahren kann das Sammeln von Daten über einen bestimmten Zeitraum einschließen. Die Proben der Patienten können zu regelmäßigen oder unregelmäßigen, bevorzugterweise regelmäßigen Zeitpunkten entnommen werden. Der Abstand zwischen der Entnahme von zwei Proben kann zwischen einer Woche und zwölf Monaten, bevorzugterweise zwischen zwei Wochen und vier Monaten und weiter bevorzugt zwischen 4 und 6 Wochen liegen, um den Titeranstieg zu verfolgen. Dieses Verfahren erlaubt den Fortschritt der *Chlamydia trachomatis*-Infektion zu beobachten.

### Beispiel 1 - Identifizierung der erfindungsgemäßen Antigene

Die Identifizierung und Charakterisierung der *Chlamydia trachomatis*-spezifischen -Antigene wurde mittels der 2D-Gelelektrophorese und Massenspektrometrie durchgeführt.

Dazu wurde zunächst eine induzierbare Genexpressionsdatenbank für *Chlamydia pneumoniae* und *Chlamydia trachomatis* in *Escherichia coli* hergestellt. Dazu wurden Seren von Patienten mit klinisch und mikrobiologisch gesicherten *Chlamydia pneumoniae* bzw. *Chlamydia trachomatis*-Infektionen gepoolt und gegen in-vitro exprimierte Chlamydien-Antigene präabsorbiert. Dazu wurden Lysate von *Chlamydia pneumoniae*-infizierten HeLa229-Zellen zu verschiedenen Infektionszeitpunkten präpariert.

Nach Kolonieplot-Analyse mit den so präabsorbierten Seren werden die reaktiven Klone isoliert und die klonierten DNA-Fragmente sequenziert. Durch Vergleich mit den bereits annotierten Sequenzen von *Chlamydia trachomatis* und *Chlamydia pneumoniae* konnte die Identität der in-vivo exprimierten Antigene geklärt werden.

Im Detail erfolgte die Identifizierung der Antigene wie folgt: *Chlamydia trachomatis* serovar D/UW-3/Cx wurde auf HeLa 229 kultiviert; 48-72h nach Infektion wurden die Zellen geerntet und homogenisiert. *Chlamydia trachomatis* wurde durch nachfolgende Zentrifugationsschritte vom Zellrückständen isoliert. Die erhaltenen Elementarkörperchen von *Chlamydia trachomatis* wurden mit Detergenzien lysiert und mit Ultraschall behandelt. Der erste Schritt der 2D-Immunelektrophorese wurde mit Ready IPG Strips, pH 3-10, 11 cm (BioRad); anschließend wurde eine SDS-PAGE in einem 10% Polyacrylamidgel durchgeführt. Die Gele wurden für Western-Blots geblottet bzw. für die nanoLC-ESI-MSMS-Analyse mit Commassie Brilliant Blue gefärbt. Die Western-Blots erfolgten mit Seren von Patienten mit einem klinischen Bild einer chronischen Infektion mit *Chlamydia trachomatis* in einer Verdünnung von 1:125. Reaktive Spots wurden aus den gefärbten und getrockneten Gelen der 2D-Immunelektrophorese ausgeschnitten, und mit der nanoLC-ESI-MSMS Methode analysiert. Dabei wurden die Proteine in den Spots mit Trypsin verdaut, entsalzt und aufkonzentriert. Die nanoLC-ESI-MSMS-Analyse wurde mit dem Esquire 3000 plus-Gerät (Bruker Daltronics) durchgeführt Die Proteine wurden mittels MS/MS ion search (Matrix Science) identifiziert und die Sequenzen mit öffentlich zugänglichen Datenbanken verglichen.

Die genomischen Sequenzen aller Antigene wurden mittels PCR und spezifischen Oligodesoxynukleotiden als Primern aus der genomischen DNA von *Chlamydia trachomatis* amplifiziert und die Amplifikate gereinigt. Die gereinigten Amplifikate wurden mit geeigneten Restriktionsendonukleasen, deren Spaltsequenzen in den PCR-Primern enthalten waren, gespalten, mittels Agarosegelelektrophorese gereinigt und in die Vektoren pUC8 bzw. pDS1, die mit denselben Restriktionsendonukleasen gespalten worden waren, ligiert. Kompetente *Escherichia coli* wurden mit den Ligationsprodukten transformiert. Positive Klone wurden isoliert und wurden mittels Restriktionsanalyse und DNA-Sequenzierung charakterisiert. Die Expression der Antigene erfolgte in *Escherichia coli.* Die Expression der Antigene wurde in der SDS-PAGE mit Färbung durch Coomassie Brilliant Blue sowie im Westem-Blot mit spezifischen humanen Seren von Patienten mit Antikörpern gegen *Chlamydia trachomatis* nachgewiesen. Die Antigene wurden anschließend mit chromatographischen Methoden gereinigt.

Tabelle 2 zeigt die Klondaten der *Chlamydia trachomatis*-Antigene.

**Tabelle 2. Klondaten für Chlamydia trachomatis-Antigene:**

| Antigen | Vektor | RE | Einschub [bp] | theoretisch [kDa] | Expression [kDa] | Expression [Menge] |
|---|---|---|---|---|---|---|
| CT017 | pDS1 | *BamHI-PstI* | 58-1299 | 46 | 48 | +++ |
| CT098 | pDS1 | *SalI-PstI* | 1-1707 | 64 | 66 | ++ |
| CT318-L1P | pDS1 | *BamHI*-*SalI* | 1-696 | 26 | 24 | +++ |
| CT431 | pUC8 | *BamHI-PstI* | 1-534 | 20 | 21 | ++ |
| CT456-TARP | pDS1 | *BamHI-SalI* | 1-3015 | 113 | >100 | ++ |
| CT603-TSAP | pDS1 | *BamHI-PstI* | 1-585 | 22 | 23 | +++ |
| CT664 | pDS1 | *BamHI-Xho*/*SalI** | 1-2487 | 93 | ~100 | + |

Die Seroreaktivität der rekombinanten exprimierten Proteine wurde mit denselben spezifischen Seren, die zur Identifizierung eingesetzt wurden, überprüft.

Für einzelne rekombinante Proteine wurde eine Epitopcharakterisierung durchgeführt, um spezies-spezifische Epitope zu identifizieren und abzugrenzen. Dies erfolgte durch Subklonierung einzelner Teilfragmente und Analyse der Seroreaktivität im Western-blot bzw. mittels in Festphasensynthese hergestellter Peptide und Analyse der Seroreaktivität im Line-Assay in Abhängigkeit von Sequenzhomologien. Die Seroreaktivität der identifizierten Antigene und spezies-spezifischen Teilfragmente bzw. Peptide wurde daraufhin im Line-Assay analysiert, um eine optimale Kombination der rekombinanten Antigene zu finden, die einerseits eine genaue Spezies-Differenzierung, andererseits eine Unterscheidung zwischen aktiven und abgelaufenen Chlamydien-Infektionen ermöglicht.

Um nachzuweisen, dass humane Antikörper gegen die Antigene CT017, CT098, CT318-L1P, CT431, CT456-TARP, CT603-TSAP und CT664, insbesondere bei chronischen Infektionen mit *Chlamydia trachomatis* auftreten, wurden die Seren von 24 Patienten mit klinischem Verdacht auf eine chronische Infektion mit *Chlamydia trachomatis,* sowie 6 Seren von gesunden Blutspendern mit Antikörpern gegen *Chlamydia trachomatis,* aber ohne Verdacht auf eine chronische Infektion mit *Chlamydia trachomatis,* verglichen.

Die Ergebnisse wurden mit den bereits beschriebenen Antigenen MOMP, OMP2, Hsp60 und MIP aus den *recom*Line Chlamydia und mit 4 weiteren bereits als serologisch relevant beschriebenen Antigenen (CT089, CT795, CT813 und CT858-CPAF) verglichen. Die Ergebnisse sind in Tabelle 3 zusammengefasst. Dabei zeigt sich, dass die Antigene CT017, CT089, CT318-L1P, CT431, CT603-TSAP und CT664 insbesondere mit Seren von Patienten mit klinischem Verdacht auf eine chronische Infektion mit *Chlamydia trachomatis* reagieren (CT017 mit 45,8 %; CT096 mit 66,7 %; CT318 mit 58,3 %; CT431 mit 29,2 %; CT603 mit 25,0 %; CT664 mit 50,0 %), aber kaum mit den Seren der gesunden Blutspender mit Antikörpern gegen *Chlamydia trachomatis,* aber ohne Verdacht auf eine chronische Infektion (CT017 mit 16,7 %; CT096 mit 16,7 %; CT318 mit 16,7 %; CT431 mit 16,7 %; CT603 mit 0,0 %; CT664 mit 0,0 %). Im Gegensatz dazu sind die bereits bekannten Antigene MOMP, OMP2, Hsp60 und MIP weniger dazu geeignet, zwischen Patienten mit klinischem Verdacht auf eine chronische Infektion mit *Chlamydia trachomatis* (MOMP mit 91,7 %, OMP2 mit 79,2 %; HSP60 mit 54,2 %; MIP mit 62,5 %; CT089 mit 70,8 %; CT795 mit 87,5 %; CT813 mit 95,8 %; CT858 mit 37,5 %) sowie gesunden Blutspendern mit Antikörpern gegen *Chlamydia trachomatis,* aber ohne Verdacht auf eine chronische Infektion mit *Chlamydia trachomatis* (MOMP mit 100,0 %; OMP2 mit 83,3 %; HSP60 mit 66,7 %; MIP mit 66,7 %; CT089 mit 33,3 %; CT795 mit 50,0 %; CT813 mit 100,0 %; CT858 mit 33,3%), zu unterscheiden. Die überraschenden vorteilhaften Ergebnisse der erfindungsgemäßen Antigene ergeben sich also aus einem Vergleich der rechten und mittleren Spalte der Tabelle 3.

Lediglich 3 Antigene erreichten bei allen für *Chlamydia trachomatis* seropositiven Proben aus beiden Gruppen eine sehr hohe Sensitivität von mindestens 90 % bezogen auf alle Proben (CT456-TARP mit 90,0 %; MOMP mit 93,3 %; CT813 mit 96,7 %). Der Wert errechnet sich beispielsweise für CT456-TARP wie folgt: 22 + 5 = 27 dividiert durch 24 + 6 = 30 → 90 %. Allerdings reagierten sowohl MOMP als auch CT813 häufiger mit den Seren der gesunden Blutspender mit Antikörpern gegen *Chlamydia trachomatis,* aber ohne Verdacht auf eine chronische Infektion (CT813 mit 100,0 %; MOMP mit 100,0 %; im Vergleich CT456-TARP mit 83,3 %), als mit den Seren von Patienten mit klinischem Verdacht auf eine chronische Infektion mit *Chlamydia trachomatis* (CT813 mit 95,8 %; MOMP mit 91,7%; CT456-TARP ebenfalls mit 95,8 %). Das bedeutet, dass CT456-TARP eine bessere Spezifität für Seren der Patienten mit klinischem Verdacht auf eine chronische Infektion mit *Chlamydia trachomatis* als die bereits bekannten Antigene MOMP und CT813 aufweist.

Die Antigene CT431, CT603 und CT664 haben zusätzlich eine geringere Kreuzreaktivität zu *Chlamydophila pneumoniae* als alle bereits bekannten Antigene.

Die diagnostische Anwendung der Antigene CT017, CT098, CT318-L1P, CT431, CT456-TARP, CT603-TSAP und CT664 ermöglicht damit eine deutlich bessere Unterscheidung zwischen Patienten mit klinischem Verdacht auf eine chronische Infektion mit *Chlamydia trachomatis* und gesunden, aber seropositiven Probanden, als dies mit den derzeit bekannten Antigenen der Fall ist.

**Tabelle 3. Serologische Charakterisierung der erfindungsgemäßen Chlamydia trachomatis-Antigene**

| **Antigen** | ***C*. *pneumoniae*-positive Seren ohne Verdacht auf eine *C*. *trachomatis-*Infektion** | | **Seren von Patienten mit klinischem Verdacht auf eine chronische *C*. *trachomatis*-Infektion** | | ***C*. *trachomatis*-positive Blutspenderseren ohne klinischem Verdacht auf eine chronische *C*. *trachomatis*-Infektion** | |
|---|---|---|---|---|---|---|
| | **n=20** | | **n=24** | | **n = 6** | |
| | **n** | **%** | **n** | **%** | **n** | **%** |
| CT017 | 4 | 20,0 | 11 | 45,8 | 1 | 16,7 |
| CT098 | 1 | 5,0 | 16 | 66,7 | 1 | 16,7 |
| CT318 | 4 | 20,0 | 14 | 58,3 | 1 | 16,7 |
| CT431 | 0 | 0,0 | 7 | 29,2 | 1 | 16,7 |
| CT456 | 3 | 15,0 | 22 | 91,7 | 5 | 83,3 |
| CT603 | 0 | 0,0 | 6 | 25,0 | 0 | 0,0 |
| CT664 | 0 | 0,0 | 12 | 50,0 | 0 | 0,0 |
| | | | | | | |
| MOMP | 1 | 5,0 | 22 | 91,7 | 6 | 100,0 |
| OMP2 | 2 | 10,0 | 19 | 79,2 | 5 | 83,3 |
| HSP60 | 3 | 15,0 | 13 | 54,2 | 4 | 66,7 |
| MIP | 3 | 15,0 | 15 | 62,5 | 4 | 66,7 |
| CT089 | 3 | 15,0 | 17 | 70,8 | 2 | 33,3 |
| CT795 | 2 | 10,0 | 21 | 87,5 | 3 | 50,0 |
| CT813 | 7 | 35,0 | 23 | 95,8 | 6 | 100,0 |
| CT858 | 1 | 5,0 | 9 | 37,5 | 2 | 33,3 |

### Beispiel 2 - Durchführung des Testverfahrens

Die Expression der Antigene erfolgte in *Escherichia coli,* angezogen in Schüttelkolben oder in einem Fermenter. Die Bakterien wurden durch Zentrifugation geerntet und mittels Detergentien und Ultraschallbehandlung lysiert. Die Antigene lagen danach zum Teil in "inclusion bodies" oder löslich vor. Die in "inclusion bodies" vorliegenden Antigene wurden mit Harnstoff solubilisiert.

Anschließend wurden die Antigene mit chromatographischen Methoden, insbesondere Anionen- und Kationenaustausch, schrittweise gereinigt. Die gereinigten Antigene wurden in empirisch gefundenen Verdünnungen auf ein Polyacrylamidgel aufgetragen und im elektrischen Feld der Größe nach aufgetrennt. Nach der Gelelektrophorese wurden die Antigene im elektrischen Feld auf eine Nitrozellulosemembran geblottet und damit auf der Membran immobilisiert.

Das Aufbringen auf die Membran kann jedoch auch direkte durch Aufsprühen auf die Nitrozellulosemembran von empirisch ermittelten Verdünnungen der gereinigten Antigene erfolgen. Die Membranen wurden nach dem Blot-Transfer mit einer proteinhaltigen Lösung behandelt, um noch nicht besetzte Proteinbindungsstellen auf der Nitrozellulosemembran abzusättigen.

Die Membranen wurden anschließend getrocknet und in Streifen geschnitten. Je ein Streifen wurde anschließend mit einem humanen Serum in einer Verdünnung von 1:250 über Nacht bei Raumtemperatur unter leichtem Schütteln inkubiert. Dabei binden die Antikörper in dem Patientenserum an die auf der Nitrozellulosemembran immobilisierten Antigene.

Die Nitrozellulosemembran-Streifen wurden anschließend 3 x gewaschen und nachfolgend für 60 min mit einem Sekundärantikörper inkubiert, der mit Meerrettich-Peroxidase konjugiert war.

Anschließend wurden die Nitrozellulosemembran-Streifen erneut 3 x gewaschen und in Folge mit einer TMB(Tetramethylbenzidin)-Lösung behandelt. An den Stellen, an denen Antigene immobilisiert waren, die mit Patientenantikörpern reagiert hatten, entstand daraufhin ein Farbniederschlag auf den Nitrozellulosemembran-Streifen. Die Streifen wurden anschließend getrocknet und die Färbungen analysiert (Tabelle 3).

### Beispiel 3 - Reaktivität von Teilsequenzen

Es wurden insgesamt 22 Peptide von je 20 Aminosäuren aus den Sequenzen der Antigene ausgewählt, synthetisiert und in zwei Experimenten im ELISA (direkte Adsorption der Peptide an die Mikrotiterplatte bzw. kovalente Anbindung an die Mikrotiterplatte) mit *Chlamydia trachomatis*-positiven Seren getestet. Die Sequenzen der Peptide, die Positionen in den Gesamtproteinen und die Ergebnisse sind in der Tabelle 4 zusammengefasst. Pro Antigen hat jeweils mindestens eines dieser Peptide mit mindestens einem Serum reagiert.

**Tabelle 4**

| **SEQ ID Nr.** | **Peptid** | **Position** | **Sequenz** | **Absorptive Anbindung (n =5)** | **kovalente Anbindung*) (n = 5)** |
|---|---|---|---|---|---|
| 8 | CT017-1 | P262 - V281 | PAVEETPVVTKTEEQKVTTV | 0 | 1 |
| 9 | CT017-2 | M348 - E367 | MESFYRDEQKKKRVLTGELE | 0 | n.d. |
| 10 | CT017-3 | V368 - D387 | VYPHIVKNNPGDYLLKNGED | 0 | 1 |
| 11 | CT098-1 | S162 - E181 | SQIDNKKIKNLDDYVGKVCE | 1 | 0 |
| 12 | CT098-2 | T252 - V271 | TWKRIRHPSEMVELNQELEV | 0 | 2 |
| 13 | CT098-3 | Q289 - V308 | QKEHNPWEDIEKKYPPGKRV | 0 | 1 |
| 14 | CT098-4 | F550 - D570 | FLVHGGDAGHDAEEESSDRD | 0 | 2 |
| 15 | CT318-1 | M001 - S020 | MTKHGKRIRGIQETYDLAKS | 1 | 1 |
| 16 | CT318-2 | I050 - T069 | IDPRKSDQQIRGSVSLPHGT | 0 | 0 |
| 17 | CT318-3 | P138 - R157 | PTPKAGTVTTDVVKTVAELR | 0 | 0 |
| 18 | CT431-1 | E005 - I024 | EMMHKLQDVIDRKLLDSRRI | 0 | 0 |
| 19 | CT431-2 | A101 - S120 | AVPGRRFATPHARIMIHQPS | 1 | 3 |
| 20 | CT431-3 | E152 - M171 | EATGQSREVIEKAIDRDMWM | 1 | n.d. |
| 21 | CT-456-1 | T103 - I122 | TSPDTSESSETSSTSSSDHI | 3 | n.d. |
| 22 | CT-456-2 | S273 - A292 | SIGGSRTSGPENTSDGAAAA | 1 | 0 |
| 23 | CT-456-3 | S454 - D473 | | 1 | 2 |
| 24 | CT-456-4 | 1569 - A588 | INTNNQTDDINTTDKDSDGA | 0 | n.d. |
| 25 | CT-456-5 | T598 - N617 | TESSSGDDSGSVSSSESDKN | 0 | 3 |
| 26 | CT-456-6 | S721 - T740 | SSGDESGGVSSPSSESNKNT | 0 | n.d. |
| 27 | CT603-1 | G006 - I025 | GRQAPDFSGKAVVCGEEKEI | 0 | n.d. |
| 28 | CT603-2 | A087 - F106 | ARNAGGIEGTEYPLLADPSF | 0 | 3 |
| 29 | CT603-3 | N172 - F191 | | 0 | n.d. |

| | | | | | |
|---|---|---|---|---|---|
| *) In der kovalenten Anbindung konnten nicht alle Peptide getestet werden, da einige nur in 8M Harnstoff gelöst werden konnten, der Harnstoff jedoch die kovalente Anbindung stört. | | | | | |

### Beispiel 4 - Serologische Charakterisierung der C. trachomatis-Antigene: Mit den Antigenen reaktive Seren

Es wurden verschiedene Seren mit einem Line Test für Chlamydia getestet und die Ergebnisse der Antigene mit den neuen Antigenen verglichen. Dabei zeigte sich, dass alle neuen Antigene eine bessere Diskriminierung zwischen Seren von Patienten mit klinisch relevanten Infektionen mit *Chlamydia trachomatis* im Vergleich zu Seren von seropositiven, aber klinisch unauffälligen Blutspendern ermöglichen. Die Ergebnisse sind in Tabelle 5 zusammengefasst.

Die in Tabelle 5 wiedergegebenen Ergebnisse entsprechen weitgehend den Ergebnissen der Tabelle 3, jedoch wurde ein Quotient der einzelnen Werte gebildet, der die Aussagekraft der Antigene verdeutlicht.

**Tabelle 5**

| **Antigen** | ***C*. *pneumoniae-*positive Seren ohne Verdacht auf eine *C*. *trachomatis-*Infektion** | | **Seren von Patienten mit klinischem Verdacht auf eine chronische *C*. *trachomatis*-Infektion** | | ***C*. *trachomatis-*positive Blutspenderseren ohne klinischem Verdacht auf eine chronische *C*. *trachomatis*-Infektion** | | **Spezifität für klinisch relevante Infektionen** |
|---|---|---|---|---|---|---|---|
| | **N = 20** | | **n = 24** | | **n = 6** | | |
| | N | % | n | % | N | % | % |
| CT017 | 4 | 20,0 | 11 | 45,8 | 1 | 16,7 | 73,3 |
| CT098 | 1 | 5,0 | 16 | 66,7 | 1 | 16,7 | 80,0 |
| CT318 | 4 | 20,0 | 14 | 58,3 | 1 | 16,7 | 77,8 |
| CT431 | 0 | 0,0 | 7 | 29,2 | 1 | 16,7 | 63,6 |
| CT456 | 3 | 15,0 | 22 | 91,7 | 5 | 83,3 | 52,4 |
| CT603 | 0 | 0,0 | 6 | 25,0 | 0 | 0,0 | 100,0 |
| | | | | | | | |
| MOMP* | 1 | 5,0 | 22 | 91,7 | 6 | 100,0 | 47,8 |
| OMP2* | 2 | 10,0 | 19 | 79,2 | 5 | 83,3 | 48,7 |
| HSP60* | 3 | 15,0 | 13 | 54,2 | 4 | 66,7 | 44,8 |
| MIP* | 3 | 15,0 | 15 | 62,5 | 4 | 66,7 | 48,4 |

| | | | | | | | |
|---|---|---|---|---|---|---|---|
| * Die Antigene MOMP, OMP2. HSP60 und MIP werden im *recom*Line Chlamydia verwendet und sind aus dem Stand der Technik bekannt. | | | | | | | |

Die Spezifität bei klinisch relevanten Infektionen wurde berechnet als Quotient aus der Prozentzahl der erkannten Seren von Patienten mit klinischem Verdacht auf eine chronische *C*. *trachomatis*-Infektion sowie der Summe der Prozentzahlen der erkannten Seren von Patienten mit klinischem Verdacht auf eine chronische *C*. *trachomatis*-Infektion und der *C*. *trachomatis*-positive Blutspenderseren ohne klinischem Verdacht auf eine chronische *C*. *trachomatis*-Infektion. Dabei haben alle neuen Antigene eine höhere Spezifität für klinisch relevante Infektionen (> 50% im Vergleich zu <50% für die bereits bekannten Antigene) als die im *recom*Line Chlamydia verwendeten bereits bekannten Antigene.

Die Antigene CT431 und CT603 haben zusätzlich eine geringere Kreuzreaktivität zu C. *pneumoniae* als alle bereits bekannten Antigene.

### Beispiel 5 - Herstellung eines Biochips

Die Antigene wurden durch ein kontaktloses Dispensierverfahren AD3050, Biodot) auf einen ca. 1 cm² großen Kunststoffchip aufgebracht, der z.B. aus Polycarbonat oder Polystyrol mit entsprechend aktivierter Oberfläche besteht. Der Abstand zwischen den einzelnen Antigendots beträgt 1,125 mm. Insgesamt können bis zu 99 Antigene auf den Biochip aufgetragen werden. Als Antigene werden die Antigene CT017, CT098, CT318-L1P, CT431, CT456-TARP, CT603-TSAP und CT664 von *Chlamydia trachomatis* auf den Biochip aufgebracht. Zusätzlich können Antigene, z.B. von *Chlamydia pneumoniae* und *Chlamydia psittaci* auf den Biochip aufgebracht werden, um den Status humanpathogener Chlamydieninfektionen abzuklären.

Außerdem können zusätzlich z.B. noch Antigene der Bakterien *Yersinia enterolitica, Salmonella enteritidis, Salmonella typhimurium, Campylobacter jejuni, Chlamydia trachomatis, Chlamydia pneumoniae, Borrelia burgdorferi, Streptococcus pyogenes,* der Viren Parvovirus B19 und Epstein-Barr-Virus sowie von humanen Autoimmunantigenen aufgetragen werden, um reaktive bzw. infektabhängige Arthritis zu diagnostizieren und von Autoimmunerkrankungen abzugrenzen. Außerdem können für weitere Fragestellungen andere Antigene oder Kombinationen von diesen mit anderen Antigenen auf den Biochip aufgetragen werden.

Der Biochip wird in eine Kunststoffkartusche eingesetzt. Die Kunststoffkartusche bildet ein abgeschlossenes System mit den Komponenten: Kartuschenkörper mit Serumbehälter und Mikrofluidkanälen, Kunststoffchip mit immobilisierten Antigenen, Deckfolie zum Versiegeln der Kartusche und elastisches Septum für die fluidische Ankopplung an einen Assayprozessor, der die erforderlichen Reagenzlösungen wie Puffer, Fluoreszenzfarbstoffkonjugate und Konditioniermedien in die BioChip-Kartusche pumpt.

Im ersten Schritt des Testablaufes wird der Serumbehälter der Kartusche mit verdünntem Serum befüllt und die Kartusche mit dem integriertem Biochip in den Assayprozessor eingesetzt und das Serum gleichmäßig über das Dot-Array gesaugt. Nach der Seruminkubation erfolgt die Reaktion gebundener Antikörper mit Fluoreszenzfarbstoffkonjugaten. Sowohl im Anschluss an die Serum- als auch an die Konjugatinkubation sorgt ein Spülzyklus für die Entfernung der nicht gebundenen Serum- bzw. Konjugatreste. Die Fluoreszenz wird anschließend quantitativ in einem Fluoreszenzreader fluoreszenzspektrometrisch ausgewertet. Durch die Verwendung von Farbstoffkonjugaten mit unterschiedlichen Immunglobulinen, z.B. Anti-human-IgG und Antihuman-IgM, können unterschiedliche Immunglobulinklassen, die in unterschiedlichen Stadien der Infektion in unterschiedlichen Mengen vorliegen, quantitativ nachgewiesen werden.

### Beispiel 6 - Durchführung des Testverfahrens mit einem Biochip

Das erfindungsgemäße Testverfahren kann auch unter Verwendung eines Biochips durchgeführt werden. Die Durchführung des Verfahrens erfolgt analog zu Beispiel 2, wobei die Immobilisierung der Antigene auf einem Biochip erfolgt.

Dabei kann die Oberfläche des Biochips, auf der die Antigene immobilisiert werden, aus einer Nitrozellulosemembran, aber auch aus aktiviertem Kunststoff (beispielsweise Polystyrol, Polycarbonat, Keramik) oder aktiviertem Glas bestehen. Die Antigene können mit einem Dispensiergerät, z.B. BioDot AD3050, automatisch direkt auf die Oberfläche des Biochips aufgesprüht werden.

Nach der Immobilisierung der Antigene auf dem Biochip werden die Biochips mit einer proteinhaltigen Lösung behandelt, um noch nicht besetzte Proteinbindungsstellen auf der Oberfläche des Biochips abzusättigen. Das weitere Testverfahren erfolgt analog der Prozessierung der Nitrozellulosemembran-Streifen, wie in Beispiel 2 beschrieben.

Der Nachweis der Antigen-Antikörper-Reaktion kann zusätzlich zur Färbung mit einem peroxidasemarkierten Sekundärantikörper und dem Farbsubstrat TMB auch mit einem Sekundärantikörper erfolgen, an den ein Fluoreszenzfarbstoff konjugiert ist. Die Fluoreszenz kann dann in einem Fluoreszenzscanner, z.B. Tecan LS 200, bestimmt werden.

## Patentansprüche

1. Verfahren zum selektiven Nachweis von *Chlamydia trachomatis* Infektionen und zur Feststellung, ob es sich um eine akute Infektion mit *Chlamydia pneumoniae* oder eine chronische Infektion mit *Chlamydia trachomatis* oder eine akute *Chlamydia trachomatis* Infektion handelt, **dadurch gekennzeichnet, dass** mindestens drei Antigene zum Nachweis von Antikörpern in Proben verwendet werden, wobei die Antigene ausgewählt sind aus der Gruppe bestehend aus: CT017, CT098, CT318-L1P, CT431, CT456-TARP, CT603-TSAP und CT664, sowie aus Fragmenten und Teilsequenzen der vorstehenden Antigene, wobei die Fragmente wenigstens 15 aufeinanderfolgende Aminosäuren der Antigene und wenigstens ein diagnostisch relevantes spezies-spezifisches Epitop aufweisen.

2. Verfahren nach Anspruch 1, **dadurch gekennzeichnet, dass** mindestens vier Antigene für das Verfahren verwendet werden.

3. Verfahren nach einem der vorangegangenen Ansprüche, **dadurch gekennzeichnet, dass** das Verfahren zur Detektion der Antikörper den Nachweis eines Antigen-/ Antikörperkomplexes umfasst.

4. Verfahren nach einem der vorangegangenen Ansprüche, **dadurch gekennzeichnet, dass** das Verfahren zur Detektion der Antikörper mittels eines ELISA (Enzyme-Linked Immunosorbent Assay) Testverfahrens, Western Blots oder Line-Assays erfolgt.

5. Verfahren zum Analysieren des Krankheitsverlaufs in vitro einer *Chlamydia trachomatis -* Infektion, **dadurch gekennzeichnet, dass** in bestimmten Zeitabständen Proben von einem Patienten genommen werden und die Proben nach dem Verfahren gemäß einem der vorangegangenen Ansprüche untersucht werden.

6. Verfahren nach einem der vorangegangenen Ansprüche, **dadurch gekennzeichnet, dass** die zu untersuchenden Proben einer Zelllyse unterzogen werden, bevor sie mit den Antigenen in Kontakt gebracht werden.

7. Testkit zum selektiven Nachweis von *Chlamydia trachomatis -* Infektionen, **dadurch gekennzeichnet, dass** das Testkit mindestens drei Antigene enthält, ausgewählt aus der Gruppe bestehend aus: CT017, CT098, CT318-L1P, CT431, CT456-TARP, CT603-TSAPund CT664.

8. Biochip, **dadurch gekennzeichnet, dass** er mindestens drei Antigene, ausgewählt aus der Gruppe bestehend aus: CT017, CT098, CT318-L1P, CT431, CT456-TARP, CT603-TSAP und CT664, für den Nachweis von *Chlamydia trachomatis* -spezifischen Antikörpern aufweist.

9. Biochip nach Anspruch 8, **dadurch gekennzeichnet, dass** die *Chlamydia trachomatis-*spezifischen Antigene auf einer Stelle der Membran des Biochips angebracht sind, die räumlich getrennt ist von anderen Stellen auf der Membran, auf der andere Antigene angebracht sind.

10. Biochip nach Anspruch 8 oder 9, **dadurch gekennzeichnet, dass** räumlich getrennt von den *Chlamydia trächomatis*-spezifischen Antigenen weitere Antigene auf der Membran angebracht sind, die einen spezifischen Nachweis gegen *Chlamydia pneumoniae* oder *Chlamydia psittaci* ermöglichen.

11. Verwendung eines Antigens zur Differentialdiagnostik einer Chlamydien-Infektion, nämlich zur Feststellung, ob es sich um eine akute Infektion mit *Chlamydia pneumoniae,* eine chronische Infektion mit *Chlamydia trachomatis* oder eine akute *Chlamydia trachomatis-*Infektion handelt, **dadurch gekennzeichnet, dass** das Antigen ausgewählt ist aus CT456-TARP, sowie Teilsequenzen dieses Antigens, die wenigstens 20 aufeinanderfolgende Aminosäuren der Sequenz mit der SEQ ID NO: 5 und wenigstens ein diagnostisch relevantes spezies-spezifisches Epitop aufweisen.

## Claims

1. A method for the selective detection of *Chlamydia trachomatis* infections and for diagnosing whether it is an acute infection with *Chlamydia pneumonia* or a chronic infection with *Chlamydia trachomatis* or an acute infection with *Chlamydia trachomatis,* **characterized in that** at least three antigens for detecting antibodies in samples are used, where the antigens are selected from the group comprising: CT017, CT098, CT318-L1P, CT431, CT456-TARP, CT603-TSAP and CT664, and from fragments and partial sequences of the above antigens, the fragments having at least 15 successive amino acids of the antigens and having at least one diagnostically relevant species-specific epitope.

2. The method as claimed in claim 1, **characterized in that** at least four antigens are used for the method.

3. The method as claimed in one of the preceding claims, **characterized in that** the method of detection of the antibodies comprises the detection of an antigen/antibody complex.

4. The method as claimed in one of the preceding claims, **characterized in that** the method of detection of the antibodies takes place by an ELISA (enzyme-linked immunosorbent assay) test method, Western blot or line assay.

5. A method for analyzing in vitro the course of a *Chlamydia trachomatis* infection, **characterized in that** at defined time intervals samples are taken from a patient and the samples are investigated by the method as claimed in one of the preceding claims.

6. The method as claimed in one of the preceding claims, **characterized in that** the samples to be investigated are submitted to cell lysis before they are brought in contact with the antigens.

7. A test kit for the selective detection of *Chlamydia trachomatis* infections, **characterized in that** the test kit contains at least three antigens, selected from the group comprising: CT017, CT098, CT318-L1P, CT431, CT456-TARP, CT603-TSAP and CT664.

8. A biochip, **characterized in that** it has at least three antigens, selected from the group comprising: CT017, CT098, CT318-L1P, CT431, CT456-TARP, CT603-TSAP and CT664, for the detection of *Chlamydia trachomatis*-specific antibodies.

9. The biochip as claimed in claim 8, **characterized in that** the *Chlamydia trachomatis*specific antigens are applied at a site of the membrane of the biochip, which is spatially separated from other sites on the membrane, on which other antigens are applied.

10. The biochip as claimed in claim 8 or 9, **characterized in that**, spatially separated from the *Chlamydia trachomatis*-specific antigens, other antigens are applied on the membrane, which permit specific detection against *Chlamydia pneumoniae* or *Chlamydia psittaci.*

11. Use of an antigen for the differential diagnosis of a chlamydial infection, namely for diagnosing whether it is an acute infection with *Chlamydia pneumonia* or a chronic infection with *Chlamydia trachomatis* or an acute infection with *Chlamydia trachomatis* **characterized in that** the antigen is selected from CT456-TARP and partial sequences of this antigen, which has at least 20 successive amino acids of the sequence with SEQ ID NO: 5 and which has at least one diagnostically relevant species-specific epitope.

## Revendications

1. Procédé de détection sélective d'infections par *Chlamydia trachomatis* et permettant de déterminer s'il s'agit d'une infection aiguë par *Chlamydia pneumoniae* ou d'une infection chronique par *Chlamydia trachomatis* ou d'une infection aiguë par *Chlamydia trachomatis,* **caractérisé en ce qu'**au moins trois antigènes sont utilisés pour la détection d'anticorps dans des échantillons, les antigènes étant choisis dans le groupe constitué par : CT017, CT098, CT318-L1 P, CT431, CT456-TARP, CT603-TSAP et CT664, ainsi que les fragments et les séquences partielles des antigènes précédents, les fragments comprenant au moins 15 acides aminés successifs des antigènes et au moins un épitope spécifique à l'espèce pertinent pour le diagnostic.

2. Procédé selon la revendication 1, **caractérisé en ce qu'**au moins quatre antigènes sont utilisés pour le procédé.

3. Procédé selon l'une quelconque des revendications précédentes, **caractérisé en ce que** le procédé de détection des anticorps comprend la détection d'un complexe antigène/anticorps.

4. Procédé selon l'une quelconque des revendications précédentes, **caractérisé en ce que** le procédé de détection des anticorps a lieu par un procédé de test ELISA (Enzyme-Linked Immunosorbent Assay), un buvardage de Western ou un essai des lignes de régression.

5. Procédé d'analyse du déroulement in vitro d'une infection par *Chlamydia trachomatis,* **caractérisé en ce que** des échantillons sont prélevés d'un patient à des intervalles temporels déterminés et les échantillons sont analysés par le procédé selon l'une quelconque des revendications précédentes.

6. Procédé selon l'une quelconque des revendications précédentes, **caractérisé en ce que** les échantillons à analyser sont soumis à une lyse cellulaire avant d'être mis en contact avec les antigènes.

7. Kit de test pour la détection sélective d'infections par *Chlamydia trachomatis,* **caractérisé en ce que** le kit de test contient au moins trois antigènes choisis dans le groupe constitué par: CT017, CT098, CT318-L1P, CT431, CT456-TARP, CT603-TSAP et CT664.

8. Biopuce, **caractérisée en ce qu'**elle comprend au moins trois antigènes choisis dans le groupe constitué par: CT017, CT098, CT318-L1P, CT431, CT456-TARP, CT603-TSAP et CT664, pour la détection d'anticorps spécifiques à *Chlamydia trachomatis.*

9. Biopuce selon la revendication 8, **caractérisée en ce que** les antigènes spécifiques à *Chlamydia trachomatis* sont appliqués à un emplacement de la membrane de la biopuce qui est séparé dans l'espace d'autres emplacements sur la membrane auxquels d'autres antigènes sont appliqués.

10. Biopuce selon la revendication 8 ou 9, **caractérisée en ce que** d'autres antigènes, qui permettent une détection spécifique de *Chlamydia pneumoniae* ou de *Chlamydia psittaci,* sont appliqués sur la membrane séparément dans l'espace des antigènes spécifiques à *Chlamydia trachomatis.*

11. Utilisation d'un antigène pour le diagnostic différentiel d'une infection par des chlamydies, à savoir pour déterminer s'il s'agit d'une infection aiguë par *Chlamydia pneumoniae,* d'une infection chronique par *Chlamydia trachomatis* ou d'une infection aiguë par *Chlamydia trachomatis,* **caractérisée en ce que** l'antigène est choisi parmi CT456-TARP, ainsi que les séquences partielles de cet antigène, qui comprennent au moins 20 acides aminés successifs de la séquence de SEQ ID NO: 5 et au moins un épitope spécifique à l'espèce pertinent pour le diagnostic.


### SEQUENCE LISTING

<110> MIKROGEN molekularbiologische Entwicklungs-GmbH
<120> verfahren, Testkit und Biochip zum selektiven Nachweis von Chlamydia trachomatis-Infektionen
<130> MIKROGEN
<160> 29
<170> PatentIn version 3.5
<210> 1
   <211> 433
   <212> PRT
   <213> Chlamydia trachomatis
<400> 1
   Met Leu Ile Phe Ala Leu Ser Phe Gly Ala Asp Ala Cys Leu Cys Ala
   1               5                   10                  15
   Ala Asp Leu Ser Lys Ala Lys Val Glu Ala Ser Val Gly Asp Arg Ala
               20                  25                  30
   Ala Phe Ser Pro Phe Thr Gly Glu Ile Lys Gly Asn Arg Val Arg Leu
           35                  40                  45
   Arg Leu Ala Pro His Thr Asp Ser Phe Ile Ile Lys Glu Leu Ser Lys
       50                  55                  60
   Gly Asp Cys Leu Ala Val Leu Gly Glu Ser Lys Asp Tyr Tyr Val Val
   65                  70                  75                  80
   Ala Ala Pro Glu Gly Val Arg Gly Tyr Val Phe Arg Thr Phe Val Leu
                   85                  90                  95
   Asp Asn Val Ile Glu Gly Glu Lys Val Asn Val Arg Leu Glu Pro Ser
               100                 105                 110
   Thr Ser Ala Pro Ile Leu Ala Arg Leu Ser Lys Gly Thr Val Val Lys
           115                 120                 125
   Thr Leu Gly Ala Ala Gln Gly Lys Trp Ile Glu Ile Ala Leu Pro Lys
       130                 135                 140
   Gln Cys Val Phe Tyr Val Ala Lys Asn Phe Val Lys Asn Val Gly Ala
   145                 150                 155                 160
   Leu Asp Leu Tyr Asn Gln Lys Glu Gly Gln Lys Lys Leu Ala Leu Asp
                   165                 170                 175
   Leu Leu Ser Ser Ala Met Asp Phe Ala Asp Ala Glu Leu Gln Lys Lys
               180                 185                 190
   Ile Glu Asp Ile Asp Leu Asp Ala Ile Tyr Lys Lys Met Asn Leu Ala
   195 200 205
   Gln Ser Glu Glu Phe Lys Asp Val Pro Gly Leu Gln Ser Leu Val Gln
   210 215 220
   Lys Ala Leu Glu Arg Val Gln Glu Ala Phe Leu Ala Lys Ser Leu Glu
   225 230 235 240
   Lys Ser Ser Val Lys Val Pro Glu Ile Arg His Lys Val Leu Glu Glu
   245 250 255
   Ile Ala Val Val Ser Pro Ala Val Glu Glu Thr Pro Val Val Thr Lys
   260 265 270
   Thr Glu Glu Gln Lys Val Thr Thr Val Pro Val Pro Ala Pro Ala Val
   275 280 285
   Val Thr Glu Pro Ala Gln Asp Leu Ser Ser Val Lys Gly Ser Leu Leu
   290 295 300
   Ser His Tyr Ile Arg Lys Lys Gly Phe Val Lys Ala Ser Pro Val Ile
   305 310 315 320
   Glu Gly Arg Glu Ser Phe Glu Arg Ser Leu Phe Ala Val Trp Leu Ser
   325 330 335
   Leu Gln Pro Glu Glu Ile Arg His Gln Leu Thr Met Glu Ser Phe Tyr
   340 345 350
   Arg Asp Glu Gln Lys Lys Lys Arg Val Leu Thr Gly Glu Leu Glu Val
   355 360 365
   Tyr Pro His Ile Val Lys Asn Asn Pro Gly Asp Tyr Leu Leu Lys Asn
   370 375 380
   Gly Glu Asp Val Val Ala Phe Val Tyr Ala Thr Ser Ile Asp Leu Ser
   385 390 395 400
   Lys Trp Leu Gly Lys Ser Val Val Leu Glu Cys Val Ser Arg Pro Asn
   405 410 415
   Asn His Phe Ala Phe Pro Ala Tyr Ile Val Leu Ser Val Lys Glu Gly
   420 425 430
   Ala
<210> 2
   <211> 569
   <212> PRT
   <213> Chlamydia trachomatis
<400> 2
   Met Pro Lys Gln Ala Asp Tyr Thr Trp Gly Ala Lys Lys Asn Leu Asp
   1               5                   10                  15
   Thr Ile Ala Cys Leu Pro Glu Asp Val Lys Gln Phe Lys Asp Leu Leu
               20                  25                  30
   Tyr Ala Met Tyr Gly Phe Thr Ala Thr Glu Glu Glu Pro Thr Ser Glu
           35                  40                  45
   Val His Pro Gly Ala Ile Leu Lys Gly Thr Val Val Asp Ile Ser Lys
       50                  55                  60
   Asp Phe Val Val Val Asp Val Gly Leu Lys Ser Glu Gly Val Ile Pro
   65                  70                  75                  80
   Met Ser Glu Phe Ile Asp Ser Ser Glu Gly Leu Thr Val Gly Ala Glu
                   85                  90                  95
   Val Glu Val Tyr Leu Asp Gln Thr Glu Asp Asp Glu Gly Lys Val Val
               100                 105                 110
   Leu Ser Arg Glu Lys Ala Thr Arg Gln Arg Gln Trp Glu Tyr Ile Leu
           115                 120                 125
   Ala His Cys Glu Glu Gly Ser Ile Val Lys Gly Gln Ile Thr Arg Lys
       130                 135                 140
   Val Lys Gly Gly Leu Ile Val Asp Ile Gly Met Glu Ala Phe Leu Pro
   145                 150                 155                 160
   Gly Ser Gln Ile Asp Asn Lys Lys Ile Lys Asn Leu Asp Asp Tyr Val
                   165                 170                 175
   Gly Lys Val Cys Glu Phe Lys Ile Leu Lys Ile Asn Val Asp Arg Arg
               180                 185                 190
   Asn Val Val Val Ser Arg Arg Glu Leu Leu Glu Ala Glu Arg Ile Ser
           195                 200                 205
   Lys Lys Ala Glu Leu Ile Glu Gln Ile Thr Ile Gly Glu Arg Arg Lys
       210                 215                 220
   Gly Ile Val Lys Asn Ile Thr Asp Phe Gly Val Phe Leu Asp Leu Asp
   225                 230                 235                 240
   Gly Ile Asp Gly Leu Leu His Ile Thr Asp Met Thr Trp Lys Arg Ile
                   245                 250                 255
   Arg His Pro Ser Glu Met Val Glu Leu Asn Gln Glu Leu Glu Val Ile
   260 265 270
   Ile Leu Ser Val Asp Lys Glu Lys Gly Arg Val Ala Leu Gly Leu Lys
   275 280 285
   Gln Lys Glu His Asn Pro Trp Glu Asp Ile Glu Lys Lys Tyr Pro Pro
   290 295 300
   Gly Lys Arg Val Arg Gly Lys Ile Val Lys Leu Leu Pro Tyr Gly Ala
   305 310 315 320
   Phe Ile Glu Ile Glu Glu Gly Ile Glu Gly Leu Ile His Val Ser Glu
   325 330 335
   Met Ser Trp Val Lys Asn Ile Val Asp Pro Asn Glu Val Val Asn Lys
   340 345 350
   Gly Asp Glu Val Glu Val Val Val Leu Ser Ile Gln Lys Asp Glu Gly
   355 360 365
   Lys Ile Ser Leu Gly Leu Lys Gln Thr Lys His Asn Pro Trp Asp Asn
   370 375 380
   Ile Glu Glu Lys Tyr Pro Ile Gly Leu Arg Val Thr Ala Glu Ile Lys
   385 390 395 400
   Asn Leu Thr Asn Tyr Gly Ala Phe Val Glu Leu Glu Pro Gly Ile Glu
   405 410 415
   Gly Leu Ile His Ile Ser Asp Met Ser Trp Ile Lys Lys Val Ser His
   420 425 430
   Pro Ser Glu Leu Phe Lys Lys Gly Asn Thr Val Glu Ala Val Ile Leu
   435 440 445
   Ser Val Asp Lys Glu Ser Lys Lys Ile Thr Leu Gly Val Lys Gln Leu
   450 455 460
   Thr Pro Asn Pro Trp Asp Glu Ile Glu Val Met Phe Pro Val Gly Ser
   465 470 475 480
   Asp Ile Ser Gly Val Val Thr Lys Ile Thr Ala Phe Gly Ala Phe Val
   485 490 495
   Glu Leu Gln Asn Gly Ile Glu Gly Leu Ile His Val Ser Glu Leu Ser
   500 505 510
   Glu Lys Pro Phe Ala Lys Ile Glu Asp Val Leu Ser Ile Gly Asp Lys
   515 520 525
   Val Ser Ala Lys Val Ile Lys Leu Asp Pro Asp His Lys Lys Val Ser
   530 535 540
   Leu Ser Ile Lys Glu Phe Leu Val His Gly Gly Asp Ala Gly His Asp
   545 550 555 560
   Ala Glu Glu Glu Ser Ser Asp Arg Asp
   565
<210> 3
   <211> 232
   <212> PRT
   <213> Chlamydia trachomatis
<400> 3
   Met Thr Lys His Gly Lys Arg Ile Arg Gly Ile Gln Glu Thr Tyr Asp
   1 5 10 15
   Leu Ala Lys Ser Tyr Ser Leu Gly Glu Ala Ile Asp Ile Leu Lys Gln
   20 25 30
   Cys Pro Thr Val Arg Phe Asp Gln Thr Val Asp Val Ser Val Lys Leu
   35 40 45
   Gly Ile Asp Pro Arg Lys Ser Asp Gln Gln Ile Arg Gly Ser Val Ser
   50 55 60
   Leu Pro His Gly Thr Gly Lys Val Leu Arg Ile Leu Val Phe Ala Ala
   65 70 75 80
   Gly Asp Lys Ala Ala Glu Ala Ile Glu Ala Gly Ala Asp Phe Val Gly
   85 90 95
   Ser Asp Asp Leu Val Glu Lys Ile Lys Gly Gly Trp Val Asp Phe Asp
   100 105 110
   Val Ala Val Ala Thr Pro Asp Met Met Arg Glu Val Gly Lys Leu Gly
   115 120 125
   Lys Val Leu Gly Pro Arg Asn Leu Met Pro Thr Pro Lys Ala Gly Thr
   130 135 140
   Val Thr Thr Asp Val Val Lys Thr Val Ala Glu Leu Arg Lys Gly Lys
   145 150 155 160
   Ile Glu Phe Lys Ala Asp Arg Ala Gly Val Cys Asn Val Gly Val Ala
   165 170 175
   Lys Leu Ser Phe Asp Ser Ala Gln Ile Lys Glu Asn Val Glu Ala Leu
   180 185 190
   Cys Ala Ala Leu Val Lys Ala Lys Pro Ala Thr Ala Lys Gly Gln Tyr
   195 200 205
   Leu Val Asn Phe Thr Ile Ser Ser Thr Met Gly Pro Gly Val Thr Val
   210 215 220
   Asp Thr Arg Glu Leu Ile Ala Leu
   225 230
<210> 4
   <211> 192
   <212> PRT
   <213> Chlamydia trachomatis
<400> 4
   Met Pro Glu Gly Glu Met Met His Lys Leu Gln Asp Val Ile Asp Arg
   1 5 10 15
   Lys Leu Leu Asp Ser Arg Arg Ile Phe Phe Ser Glu Pro Val Thr Glu
   20 25 30
   Lys Ser Ala Thr Glu Ala Ile Lys Lys Leu Trp Tyr Leu Glu Leu Thr
   35 40 45
   Asn Pro Gly Gln Pro Ile Val Phe Val Ile Asn Ser Pro Gly Gly Ser
   50 55 60
   Val Asp Ala Gly Phe Ala Val Trp Asp Gln Ile Lys Met Ile Ser Ser
   65 70 75 80
   Pro Leu Thr Thr Val Val Thr Gly Leu Ala Ala Ser Met Gly Ser Val
   85 90 95
   Leu Ser Leu Cys Ala Val Pro Gly Arg Arg Phe Ala Thr Pro His Ala
   100 105 110
   Arg Ile Met Ile His Gln Pro Ser Ile Gly Gly Thr Ile Thr Gly Gln
   115 120 125
   Ala Thr Asp Leu Asp Ile His Ala Arg Glu Ile Leu Lys Thr Lys Ala
   130 135 140
   Arg Ile Ile Asp Val Tyr Val Glu Ala Thr Gly Gln Ser Arg Glu Val
   145 150 155 160
   Ile Glu Lys Ala Ile Asp Arg Asp Met Trp Met Ser Ala Asn Glu Ala
   165 170 175
   Met Glu Phe Gly Leu Leu Asp Gly Ile Leu Phe Ser Phe Asn Asp Leu
   180 185 190
<210> 5
   <211> 1005
   <212> PRT
   <213> Chlamydia trachomatis
<400> 5
   Met Thr Asn Ser Ile Ser Gly Tyr Gln Pro Thr Val Thr Thr Ser Thr
   1               5                   10                  15
   Ser Ser Thr Thr Ser Ala Ser Gly Ala Ser Gly Ser Leu Gly Ala Ser
               20                  25                  30
   Ser Val Ser Thr Thr Ala Asn Ala Thr Val Thr Gln Thr Ala Asn Ala
           35                  40                  45
   Thr Asn Ser Ala Ala Thr Ser Ser Ile Gln Thr Thr Gly Glu Thr Val
       50                  55                  60
   Val Asn Tyr Thr Asn Ser Ala Ser Ala Pro Asn Val Thr Val Ser Thr
   65                  70                  75                  80
   Ser Ser Ser Ser Thr Gln Ala Thr Ala Thr Ser Asn Lys Thr Ser Gln
                   85                  90                  95
   Ala Val Ala Gly Lys Ile Thr Ser Pro Asp Thr Ser Glu Ser Ser Glu
               100                 105                 110
   Thr Ser Ser Thr Ser Ser Ser Asp His Ile Pro Ser Asp Tyr Asp Asp
           115                 120                 125
   Val Gly Ser Asn Ser Gly Asp Ile Ser Asn Asn Tyr Asp Asp Val Gly
       130                 135                 140
   Ser Asn Asn Gly Asp Ile Ser Ser Asn Tyr Asp Asp Ala Ala Ala Asp
   145                 150                 155                 160
   Tyr Glu Pro Ile Arg Thr Thr Glu Asn Ile Tyr Glu Ser Ile Gly Gly
                   165                 170                 175
   Ser Arg Thr Ser Gly Pro Glu Asn Thr Ser Gly Gly Ala Ala Ala Ala
               180                 185                 190
   Leu Asn Ser Leu Arg Gly Ser Ser Tyr Ser Asn Tyr Asp Asp Ala Ala
           195                 200                 205
   Ala Asp Tyr Glu Pro Ile Arg Thr Thr Glu Asn Ile Tyr Glu Ser Ile
       210                 215                 220
   Gly Gly Ser Arg Thr Ser Gly Pro Glu Asn Thr Ser Gly Gly Ala Ala
   225                 230                 235                 240
   Ala Ala Leu Asn Ser Leu Arg Gly Ser Ser Tyr Ser Asn Tyr Asp Asp
                   245                 250                 255
   Ala Ala Ala Asp Tyr Glu Pro Ile Arg Thr Thr Glu Asn Ile Tyr Glu
   260 265 270
   Ser Ile Gly Gly Ser Arg Thr Ser Gly Pro Glu Asn Thr Ser Asp Gly
   275 280 285
   Ala Ala Ala Ala Ala Leu Asn Ser Leu Arg Gly Ser Ser Tyr Thr Thr
   290 295 300
   Gly Pro Arg Asn Glu Gly Val Phe Gly Pro Gly Pro Glu Gly Leu Pro
   305 310 315 320
   Asp Met Ser Leu Pro Ser Tyr Asp Pro Thr Asn Lys Thr Ser Leu Leu
   325 330 335
   Thr Phe Leu Ser Asn Pro His Val Lys Ser Lys Met Leu Glu Asn Ser
   340 345 350
   Gly His Phe Val Phe Ile Asp Thr Asp Arg Ser Ser Phe Ile Leu Val
   355 360 365
   Pro Asn Gly Asn Trp Asp Gln Val Cys Ser Ile Lys Val Gln Asn Gly
   370 375 380
   Lys Thr Lys Glu Asp Leu Asp Ile Lys Asp Leu Glu Asn Met Cys Ala
   385 390 395 400
   Lys Phe Cys Thr Gly Phe Ser Lys Phe Ser Gly Asp Trp Asp Ser Leu
   405 410 415
   Val Glu Pro Met Val Ser Ala Lys Ala Gly Val Ala Ser Gly Gly Asn
   420 425 430
   Leu Pro Asn Thr Val Ile Ile Asn Asn Lys Phe Lys Thr Cys Val Ala
   435 440 445
   Tyr Gly Pro Trp Asn Ser Gln Glu Ala Ser Ser Gly Tyr Thr Pro Ser
   450 455 460
   Ala Trp Arg Arg Gly His Arg Val Asp Phe Gly Gly Ile Phe Glu Lys
   465 470 475 480
   Ala Asn Asp Phe Asn Lys Ile Asn Trp Gly Thr Gln Ala Gly Pro Ser
   485 490 495
   Ser Glu Asp Asp Gly Ile Ser Phe Ser Asn Glu Thr Pro Gly Ala Gly
   500 505 510
   Pro Ala Ala Ala Pro Ser Pro Thr Pro Ser Ser Ile Pro Ile Ile Asn
   515 520 525
   Val Asn Val Asn Val Gly Gly Thr Asn Val Asn Ile Gly Asp Thr Asn
   530 535 540
   Val Asn Thr Thr Asn Thr Thr Pro Thr Thr Gln Ser Thr Asp Ala Ser
   545 550 555 560
   Thr Asp Thr Ser Asp Ile Asp Asp Ile Asn Thr Asn Asn Gln Thr Asp
   565 570 575
   Asp Ile Asn Thr Thr Asp Lys Asp Ser Asp Gly Ala Gly Gly Val Asn
   580 585 590
   Gly Asp Ile Ser Glu Thr Glu Ser Ser Ser Gly Asp Asp Ser Gly Ser
   595 600 605
   Val Ser Ser Ser Glu Ser Asp Lys Asn Ala Ser Val Gly Asn Asp Gly
   610 615 620
   Pro Ala Met Lys Asp Ile Leu Ser Ala Val Arg Lys His Leu Asp Val
   625 630 635 640
   Val Tyr Pro Gly Glu Asn Gly Gly Ser Thr Glu Gly Pro Leu Pro Ala
   645 650 655
   Asn Gln Thr Leu Gly Asp Val Ile Ser Asp Val Glu Asn Lys Gly Ser
   660 665 670
   Ala Gln Asp Thr Lys Leu Ser Gly Asn Thr Gly Ala Gly Asp Asp Asp
   675 680 685
   Pro Thr Thr Thr Ala Ala Val Gly Asn Gly Ala Glu Glu Ile Thr Leu
   690 695 700
   Ser Asp Thr Asp Ser Gly Ile Gly Asp Asp Val Ser Asp Thr Ala Ser
   705 710 715 720
   Ser Ser Gly Asp Glu Ser Gly Gly Val Ser Ser Pro Ser Ser Glu Ser
   725 730 735
   Asn Lys Asn Thr Ala Val Gly Asn Asp Gly Pro Ser Gly Leu Asp Ile
   740 745 750
   Leu Ala Ala Val Arg Lys His Leu Asp Lys Val Tyr Pro Gly Asp Asn
   755 760 765
   Gly Gly Ser Thr Glu Gly Pro Leu Gln Ala Asn Gln Thr Leu Gly Asp
   770 775 780
   Ile Val Gln Asp Met Glu Thr Thr Gly Thr Ser Gln Glu Thr Val Val
   785 790 795 800
   Ser Pro Trp Lys Gly Ser Thr Ser Ser Thr Glu Ser Ala Gly Gly Ser
   805 810 815
   Gly Ser Val Gln Thr Leu Leu Pro Ser Pro Pro Pro Thr Pro Ser Thr
   820 825 830
   Thr Thr Leu Arg Thr Gly Thr Gly Ala Thr Thr Thr Ser Leu Met Met
   835 840 845
   Gly Gly Pro Ile Lys Ala Asp Ile Ile Thr Thr Gly Gly Gly Gly Arg
   850 855 860
   Ile Pro Gly Gly Gly Thr Leu Glu Lys Leu Leu Pro Arg Ile Arg Ala
   865 870 875 880
   His Leu Asp Ile Ser Phe Asp Ala Gln Gly Asp Leu Val Ser Thr Glu
   885 890 895
   Glu Pro Gln Leu Gly Ser Ile Val Asn Lys Phe Arg Gln Glu Thr Gly
   900 905 910
   Ser Arg Gly Ile Leu Ala Phe Val Glu Ser Ala Pro Gly Lys Pro Gly
   915 920 925
   Ser Ala Gln Val Leu Thr Gly Thr Gly Gly Asp Lys Gly Asn Leu Phe
   930 935 940
   Gln Ala Ala Ala Ala Val Thr Gln Ala Leu Gly Asn Val Ala Gly Lys
   945 950 955 960
   Val Asn Leu Ala Ile Gln Gly Gln Lys Leu Ser Ser Leu Val Asn Asp
   965 970 975
   Asp Gly Lys Gly Ser Val Gly Arg Asp Leu Phe Gln Ala Ala Ala Gln
   980 985 990
   Thr Thr Gln Val Leu Ser Ala Leu Ile Asp Thr Val Gly
   995 1000 1005
<210> 6
   <211> 195
   <212> PRT
   <213> Chlamydia trachomatis
<400> 6
   Met Gly Ser Leu Val Gly Arg Gln Ala Pro Asp Phe Ser Gly Lys Ala
   1 5 10 15
   Val Val Cys Gly Glu Glu Lys Glu Ile Ser Leu Ala Asp Phe Arg Gly
   20 25 30
   Lys Tyr Val Val Leu Phe Phe Tyr Pro Lys Asp Phe Thr Tyr Val Cys
   35 40 45
   Pro Thr Glu Leu His Ala Phe Gln Asp Arg Leu Val Asp Phe Glu Glu
   50 55 60
   Arg Gly Ala Val Val Leu Gly Cys Ser Val Asp Asp Ile Glu Thr His
   65 70 75 80
   Ser Arg Trp Leu Ala Val Ala Arg Asn Ala Gly Gly Ile Glu Gly Thr
   85 90 95
   Glu Tyr Pro Leu Leu Ala Asp Pro Ser Phe Lys Ile Ser Glu Ala Phe
   100 105 110
   Gly Val Leu Asn Pro Glu Gly Ser Leu Ala Leu Arg Ala Thr Phe Leu
   115 120 125
   Ile Asp Lys Tyr Gly Val Val Arg His Ala Val Ile Asn Asp Leu Pro
   130 135 140
   Leu Gly Arg Ser Ile Asp Glu Glu Leu Arg Ile Leu Asp Ser Leu Ile
   145 150 155 160
   Phe Phe Glu Asn His Gly Met Val Cys Pro Ala Asn Trp Arg Ser Gly
   165 170 175
   Glu Arg Gly Met Val Pro Ser Glu Glu Gly Leu Lys Glu Tyr Phe Gln
   180 185 190
   Thr Met Asp
   195
<210> 7
   <211> 829
   <212> PRT
   <213> chlamydia trachomatis
<400> 7
   Met Gly Ile Arg Leu Val Ile Asp Lys Gly Pro Leu Ser Gly Thr Val
   1 5 10 15
   Leu Ile Leu Glu Asn Gly Thr Ser Trp Ser Leu Gly Ser Asp Gly Lys
   20 25 30
   Ala Ser Asp Ile Leu Leu Gln Asp Glu Lys Leu Ala Pro Ser Gln Ile
   35 40 45
   Arg Ile Thr Leu Lys Asp Gly Glu Tyr Tyr Leu Glu Asn Leu Asp Ala
   50 55 60
   Leu Arg Pro Val Ser Val Asp Gly Thr Val Ile Thr Ala Pro Val Leu
   65 70 75 80
   Leu Lys Asp Gly Val Ser Phe Val Met Gly Ser Cys Gln Val Ser Phe
   85 90 95
   Phe Lys Gly Glu Glu Val Glu Gly Asp Ile Glu Leu Ser Phe Gln Thr
   100 105 110
   Glu Gly Gly Asn Glu Gly Glu Pro Ala Ala Gln Gly Ser Ser Ser Val
   115 120 125
   Ser Ser Glu Ala Pro Lys Lys Glu Thr Gly Asn Pro Ser Leu Pro Ser
   130 135 140
   Glu Ala Lys Ala Ser Gly Glu Val Ser Ser Ser Ala Ile Ala Lys Glu
   145 150 155 160
   Gln Glu Leu Ala Ala Ser Phe Leu Ala Ser Val Glu Lys Glu Pro Gly
   165 170 175
   Thr Pro Lys Glu Val Ser Glu Pro Lys Val Ser Ser Gln Glu Gly Gln
   180 185 190
   Thr Pro Ser Val Thr Gly Glu Lys Lys Asp Leu Glu Leu Pro Leu Ala
   195 200 205
   Ser Gln Glu Gln Pro Lys Gln Thr Thr Pro Ser Gly Ser Gly Glu Pro
   210 215 220
   Thr Gln Ser Gln Asn Ala Ser Met Glu Glu Asn Arg Thr Ser Pro Asp
   225 230 235 240
   Gln Asn Gln Gln Pro Gln Leu Ser Ser Ala Ser Glu Ser Gly Ser Gln
   245 250 255
   Ser Pro Glu Asn Gln Glu Gln Gln Pro Ser Gln Thr Pro Pro Pro Ser
   260 265 270
   Pro Glu Thr Pro Glu Pro Ser Gly Glu Pro Asn Ser Ala Thr Glu Glu
   275 280 285
   Asn Ser Pro Ser Pro Met Glu Lys Ala Ser Val Thr Glu Glu Gly Ser
   290 295 300
   Ser Gly Thr Ser Glu Glu Glu Lys Glu Gly Glu Glu Asp Thr Ala Glu
   305 310 315 320
   Ser Ala Ala Asn Glu Glu Pro Lys Ala Glu Ala Ser Gln Glu Glu Glu
   325 330 335
   Lys Lys Glu Glu Asp Lys Gly Glu Val Leu Ala Pro Phe Asn Val Gln
   340 345 350
   Asp Leu Phe Arg Phe Asp Gln Gly Ile Phe Pro Ala Glu Ile Glu Asp
           355                 360                 365
   Leu Ala Gln Lys Gln Val Ala Val Asp Leu Thr Gln Pro Ser Arg Phe
       370                 375                 380
   Leu Leu Lys Val Leu Ala Gly Ala Asn Ile Gly Ala Glu Phe His Leu
   385                 390                 395                 400
   Asp Ser Gly Lys Thr Tyr Ile Val Gly Ser Asp Pro Gln Val Ala Asp
                   405                 410                 415
   Ile Val Leu Ser Asp Met Ser Ile Ser Arg Gln His Ala Lys Ile Ile
               420                 425                 430
   Ile Gly Asn Asp Asn Ser Val Leu Ile Glu Asp Leu Gly Ser Lys Asn
           435                 440                 445
   Gly Val Ile Val Glu Gly Arg Lys Ile Glu His Gln Ser Thr Leu Ser
       450                 455                 460
   Ala Asn Gln Val Val Ala Leu Gly Thr Thr Leu Phe Leu Leu Val Asp
   465                 470                 475                 480
   Tyr Ala Ala Pro Ser Asp Thr Val Met Ala Thr Ile Ser Ser Glu Asp
                   485                 490                 495
   Tyr Gly Leu Phe Gly Arg Pro Gln Ser Pro Glu Glu Ile Ala Ala Arg
               500                 505                 510
   Ala Ala Glu Glu Glu Glu Glu Lys Arg Lys Arg Ala Thr Leu Pro Thr
           515                 520                 525
   Gly Ala Phe Ile Leu Thr Leu Phe Ile Gly Gly Leu Ala Leu Leu Phe
       530                 535                 540
   Gly Ile Gly Thr Ala Ser Leu Phe His Thr Lys Glu Val Val Ser Ile
   545                 550                 555                 560
   Asp Gln Ile Asp Leu Ile His Asp Ile Glu His Val Ile Gln Gln Phe
                   565                 570                 575
   Pro Thr Val Arg Phe Thr Phe Asn Lys Asn Asn Gly Gln Leu Phe Leu
               580                 585                 590
   Ile Gly His Val Arg Asn Ser Ile Asp Lys Ser Glu Leu Leu Tyr Lys
           595                 600                 605
   Val Asp Ala Leu Ser Phe Val Lys Ser Val Asp Asp Asn Val Ile Asp
       610                 615                 620
   Asp Glu Ala Val Trp Gln Glu Met Asn Ile Leu Leu Ser Lys Asn Pro
   625 630 635 640
   Glu Phe Lys Gly Ile Ser Met Gln Ser Pro Glu Pro Gly Ile Phe Val
   645 650 655
   Ile Ser Gly Tyr Leu Lys Thr Glu Glu Gln Ala Ala Cys Leu Ala Asp
   660 665 670
   Tyr Leu Asn Leu His Phe Asn Tyr Leu Ser Leu Leu Asp Asn Lys Val
   675 680 685
   Ile Ile Glu Ser Gln Val Met Lys Ala Leu Ala Gly His Leu Val Gln
   690 695 700
   Ser Gly Phe Ala Asn Val His Val Ser Phe Thr Asn Gly Glu Ala Val
   705 710 715 720
   Leu Thr Gly Tyr Ile Asn Asn Lys Asp Ala Asp Lys Phe Arg Thr Val
   725 730 735
   Val Gln Glu Leu Gln Asp Ile Ala Gly Ile Arg Ala Val Lys Asn Phe
   740 745 750
   Val Val Leu Leu Pro Ala Glu Glu Gly Val Ile Asp Leu Asn Met Arg
   755 760 765
   Tyr Pro Gly Arg Tyr Arg Val Thr Gly Phe Ser Lys Cys Gly Asp Ile
   770 775 780
   Ser Ile Asn Val Val Val Asn Gly Arg Ile Leu Thr Arg Gly Asp Ile
   785 790 795 800
   Leu Asp Gly Met Thr Val Thr Ser Ile Gln Ser His Cys Ile Phe Leu
   805 810 815
   Glu Arg Glu Gly Leu Lys Tyr Lys Ile Glu Tyr Asn Lys
   820 825
<210> 8
   <211> 20
   <212> PRT
   <213> Artificial Sequence
<220>
   <223> peptide
<400> 8
   Pro Ala Val Glu Glu Thr Pro Val Val Thr Lys Thr Glu Glu Gln Lys
   1 5 10 15
   Val Thr Thr Val
   20
<210> 9
   <211> 20
   <212> PRT
   <213> artifcial
<400> 9
   Met Glu Ser Phe Tyr Arg Asp Glu Gln Lys Lys Lys Arg Val Leu Thr
   1 5 10 15
   Gly Glu Leu Glu
   20
<210> 10
   <211> 20
   <212> PRT
   <213> artificial
<220>
   <223> peptide
<400> 10
   Val Tyr Pro His Ile Val Lys Asn Asn Pro Gly Asp Tyr Leu Leu Lys
   1 5 10 15
   Asn Gly Glu Asp
   20
<210> 11
   <211> 20
   <212> PRT
   <213> artifical
<400> 11
   Ser Gln Ile Asp Asn Lys Lys Ile Lys Asn Leu Asp Asp Tyr Val Gly
   1 5 10 15
   Lys Val Cys Glu
   20
<210> 12
   <211> 20
   <212> PRT
   <213> artificial
<220>
   <223> peptide
<400> 12
   Thr Trp Lys Arg Ile Arg His Pro Ser Glu Met Val Glu Leu Asn Gln
   1 5 10 15
   Glu Leu Glu Val
   20
<210> 13
   <211> 20
   <212> PRT
   <213> artificial
<220>
   <223> peptide
<400> 13
   Gln Lys Glu His Asn Pro Trp Glu Asp Ile Glu Lys Lys Tyr Pro Pro
   1 5 10 15
   Gly Lys Arg Val
   20
<210> 14
   <211> 20
   <212> PRT
   <213> artificial
<220>
   <223> peptide
<400> 14
   Phe Leu Val His Gly Gly Asp Ala Gly His Asp Ala Glu Glu Glu Ser
   1 5 10 15
   Ser Asp Arg Asp
   20
<210> 15
   <211> 20
   <212> PRT
   <213> artificial
<220>
   <223> peptide
<400> 15
   Met Thr Lys His Gly Lys Arg Ile Arg Gly Ile Gln Glu Thr Tyr Asp
   1 5 10 15
   Leu Ala Lys Ser
   20
<210> 16
   <211> 20
   <212> PRT
   <213> artificial
<220>
   <223> peptide
<400> 16
   Ile Asp Pro Arg Lys Ser Asp Gln Gln Ile Arg Gly Ser Val Ser Leu
   1 5 10 15
   Pro His Gly Thr
   20
<210> 17
   <211> 20
   <212> PRT
   <213> artificial
<220>
   <223> peptide
<400> 17
   Pro Thr Pro Lys Ala Gly Thr Val Thr Thr Asp Val Val Lys Thr Val
   1 5 10 15
   Ala Glu Leu Arg
   20
<210> 18
   <211> 20
   <212> PRT
   <213> artificial
<220>
   <223> peptide
<400> 18
   Glu Met Met His Lys Leu Gln Asp Val Ile Asp Arg Lys Leu Leu Asp
   1 5 10 15
   Ser Arg Arg Ile
   20
<210> 19
   <211> 20
   <212> PRT
   <213> artificial
<220>
   <223> peptide
<400> 19
   Ala Val Pro Gly Arg Arg Phe Ala Thr Pro His Ala Arg Ile Met Ile
   1               5                   10                  15
   His Gln Pro Ser
               20
<210> 20
   <211> 20
   <212> PRT
   <213> artificial
<220>
   <223> peptide
<400> 20
   Glu Ala Thr Gly Gln Ser Arg Glu Val Ile Glu Lys Ala Ile Asp Arg
   1               5                   10                  15
   Asp Met Trp Met
               20
<210> 21
   <211> 20
   <212> PRT
   <213> artificial
<220>
   <223> peptide
<400> 21
   Thr Ser Pro Asp Thr Ser Glu Ser Ser Glu Thr Ser Ser Thr Ser Ser
   1               5                   10                  15
   Ser Asp His Ile
               20
<210> 22
   <211> 20
   <212> PRT
   <213> artificial
<220>
   <223> peptide
<400> 22
   Ser Ile Gly Gly Ser Arg Thr Ser Gly Pro Glu Asn Thr Ser Asp Gly
   1               5                   10                  15
   Ala Ala Ala Ala
               20
<210> 23
   <211> 20
   <212> PRT
   <213> artificial
<220>
   <223> peptide
<400> 23
   Ser Gln Glu Ala Ser Ser Gly Tyr Thr Pro Ser Ala Trp Arg Arg Gly
   1 5 10 15
   His Arg Val Asp
   20
<210> 24
   <211> . 20
   <212> PRT
   <213> artificial
<220>
   <223> peptide
<400> 24
<210> 25
   <211> 20
   <212> PRT
   <213> artificial
<220>
   <223> peptide
<400> 25
   Thr Glu Ser Ser Ser Gly Asp Asp Ser Gly Ser Val Ser Ser Ser Glu
   1 5 10 15
   Ser Asp Lys Asn
   20
<210> 26
   <211> 20
   <212> PRT
   <213> artificial
<220>
   <223> peptide
<400> 26
   Ser Ser Gly Asp Glu Ser Gly Gly Val Ser Ser Pro Ser Ser Glu Ser
   1 5 10 15
   Asn Lys Asn Thr
   20
<210> 27
   <211> 20
   <212> PRT
   <213> artificial
<220>
   <223> peptide
<400> 27
   Gly Arg Gln Ala Pro Asp Phe Ser Gly Lys Ala Val Val Cys Gly Glu
   1 5 10 15
   Glu Lys Glu Ile
   20
<210> 28
   <211> 20
   <212> PRT
   <213> artificial
<220>
   <223> peptide
<400> 28
   Ala Arg Asn Ala Gly Gly Ile Glu Gly Thr Glu Tyr Pro Leu Leu Ala
   1 5 10 15
   Asp Pro Ser Phe
   20
<210> 29
   <211> 20
   <212> PRT
   <213> artificial
<220>
   <223> peptide
<400> 29
   Asn Trp Arg Ser Gly Glu Arg Gly Met Val Pro Ser Glu Glu Gly Leu
   1 5 10 15
   Lys Glu Tyr Phe
   20